# DEMANDE DE BREVET EUROPEEN

(11) **EP 0 743 364 A2**
(43) Date de publication de la demande: **20.11.1996**
(21) Numéro de dépôt: 96401084.7
(22) Date de dépôt: 17.05.1996
(51) Int. Cl.: C12N 15/48, C07K 14/16, C12Q 1/68, G01N 33/53

(54) **Fragments d'acide nucléique dérivés du génome du virus VIH-1, peptides correspondants et leurs applications en tant que réactifs d'évaluation du risque de transmission materno-foetal du VIH-1**

(30) Priorité: 18.05.1995 FR 9505914
(71) Demandeur: COMMISSARIAT A L'ENERGIE ATOMIQUE, 75015 Paris Cédex 15 (FR)
(72) Inventeur: Narwa, Rémy, 75020 Paris (FR); Roques, Pierre, 92160 Antony (FR)
(74) Mandataire: Orès, Bernard

(57) **Abrégé**

Fragments d'acide nucléique dérivés du génome du virus de l'immunodéflcience humaine de type 1 (VIH-1) ainsi que peptides correspondants et leurs applications en tant que réactifs de dépistage et d'évaluation du risque de transmission materno-foetale du VIH- 1.

Lesdits fragments d'acide nucléique, issus du gène codant pour un fragment de la protéine de matrice p17 du VIH-1, sont essentiellement constitués de 21 à 90 nucléotides et incluent au moins la séquence représentée par la formule : Y5 Y6 Y7 Y8 Y9 Y10 Y11, dans laquelle : Y5 représente AAA, AAG, GAA, Y6 représente ATA, TTA, CTG, GTA, CTA, GTG, ATG, Y7 représente GAG, GAA, Y8 représente GAA, AAG, Y9 représente ATA, GTA, CTA, GAA, GAG, GAC, GAT, AGA, Y10 représente CAA, CAG, CGA, Y11 représente AAA, AGT, AAG, AAT, CAT, AAC, ACC.

Lesdits peptides, constitués de 7 à 30 amino-acides et incluant au moins la séquence en aminoacides 103 à 109 de la protéine de matrice virale p17 du VIH-1, sont représentés par la formule X2 X3 Glu X4 X5 X6 X7, dans laquelle : X2 représente Lys, Glu, X3 représente IIe, Leu, Val, Met, X4 représente Glu, Lys, X5 représente IIe, Val, Glu, Arg, Leu, X6 représente Gln, Arg et X7 représente Lys, Ser, Asn, His, Thr.

## Description

La présente invention est relative à des fragments d'acide nucléique dérivés du génome du virus de l'immunodéficience humaine de type 1 (VIH-1) ainsi qu'aux peptides correspondants et à leurs applications en tant que réactifs de dépistage et d'évaluation du risque de transmission materno-foetale du VIH-1.

Deux types distincts de VIH (virus de l'immunodéficience humaine : VIH-1 et VIH-2) ont été décrits et sont les agents responsables du SIDA. L'analyse de leur séquence d'acide nucléique a permis d'identifier différents sous-types de VIH-1, bien qu'aucune corrélation entre la variabilité et la pathogénicité n'ait pu être établie.

L'analyse de fragments nucléotidiques de différents isolats de VIH-1, a montré l'existence, à travers l'analyse du gène env, d'au moins 7 sous-types différents, dénommés A à G (MYERS G. et al., Human retroviruses and AIDS, 1993, Los Alamos Nat. Lab.).

Plus récemment, deux autres isolats, considérés comme nettement plus distants des 7 autres sous-types, c'est-à-dire dont l'homologie de séquence est la plus distante de celle des souches de références VIH-1, ont également été isolés et ont été rattachés à un nouveau groupe de VIH-1, le groupe O, par opposition au groupe M correspondant aux 7 sous-types A-G précités, compte-tenu de leur organisation génomique (5' LTR Gag Pol Vif Vpu Vpr Tat Rev Env Nef LTR 3').

Compte-tenu du nombre de sujets atteints par le VIH-1 et du mode de transmission de ce virus (sexuel et sanguin), il était urgent de disposer aussi bien de réactifs de diagnostic que de compositions immunogènes aptes à induire la formation d'anticorps neutralisants.

Un certain nombre de peptides ont été proposés dans ce but, issus notamment de la protéine env ou de la protéine gag.

En ce qui concerne la protéine gag et plus particulièrement la protéine p17 (incluse dans la protéine p55), l'Art antérieur fait état d'un certain nombre de peptides qui ont été sélectionnés dans ladite protéine p17 du VIH-1, pour leur valeur diagnostic et/ou vaccinale.

On peut citer par exemple :
- la Demande Internationale PCT WO 91/08227 (REPLICO AB) qui concerne des peptides issus de la protéine p17 du VIH-1 et qui permettent essentiellement de discriminer les faux positifs des vrais positifs. Ces peptides comprennent les épitopes présents en positions 118-132 de la protéine p17 du VIH-1 (position C-terminale) ;
- le Brevet US 5,185,147 (L.D. PAPSIDERO) fait état de petits peptides (11 aminoacides ou moins), qui sont les seuls à réagir avec des anticorps monoclonaux, capables de neutraliser l'activité biologique du VIH-1 et qui sont intéressants dans un but diagnostic ou vaccinal (propriétés immunogènes). Ces différents peptides correspondent aux positions 12-22 de la protéine p17.
- La Demande de Brevet Européen 0 426 314 (VIRAL TECHNOLOGIES INC et THE GEORGE WASHINGTON UNIVERSITY) fait état de 5 segments majeurs de la protéine p17 du VIH-1, présentant des épitopes immunogènes potentiellement importants : peptide A (1-32), peptide B (33-50), peptide C (51-84), peptide D (85-114) et peptide E (114-131) et préconise l'utilisation des peptides A, B, C et E pour la détection du VIH-1 et l'utilisation des peptides A, B, C, D et E pour la préparation d'une composition immunogène.

Toutefois, l'ensemble des peptides de l'Art antérieur ne résolvent pas le problème crucial de l'évaluation du risque de transmission materno-foetale du VIH-1.

Or, en 1995, la transmission materno-foetale du VIH-1 constitue un problème majeur de santé publique, car sa progression va de pair avec celle de la contamination par le VIH-1 dans le monde.

L'Organisation Mondiale de la Santé (OMS) estime à plus d'un million, le nombre mondial d'enfants contaminés.

En France, on estime à 30 000-40 000, le nombre de femmes séropositives pour le VIH-1, en âge de procréer.

Le taux de transmission verticale est de 15 à 25 % aussi bien en Europe qu'aux Etats-Unis. Ce taux est supérieur sur le continent africain, allant de 20 à 40 % pour des raisons peu claires, sans doute en raison de co-infection de la mère et de l'allaitement maternel.

La mise sous Retrovir® (AZT) des femmes enceintes européennes et américaines, jusqu'à l'accouchement, a permis de réduire le taux de transmission à 8,3 %.

Douze ans après la découverte du virus, la méconnaissance de la physiopathologie de la transmission verticale est encore importante: de nombreux arguments indiquent que la transmission du virus de la mère au foetus peut se produire tardivement *in utero* (dans les 2 derniers mois), au moment de l'accouchement avec une fréquence probablement plus forte et au cours de l'allaitement maternel, qui est en conséquence fortement déconseillé.

Parmi les paramètres pouvant influer sur la transmission verticale, peu sont actuellement démontrés comme corrélés positivement avec le risque de transmission. Il s'agit :
- d'un stade avancé de la maladie chez la mère (grade IV),
- d'une antigénémie P24 positive, et/ou d'un taux de lymphocytes CD4+ inférieur à 200/mm³,
- d'une virémie plasmatique élevée chez la mère.

L'absence d'un de ces paramètres n'exclut cependant pas le risque de transmission verticale, il ne fait que le diminuer.

En revanche, la présence d'anticorps neutralisants chez la mère, protecteurs du passage à l'enfant, reste très controversée.

Parmi les facteurs virologiques d'expression biologique (taux de réplication, tropisme cellulaire, cytotoxicité, capacité d'induction de syncytia) ou moléculaires (notamment dans le gène codant pour la glycoprotéine d'enveloppe gp120), aucun n'a, à ce jour, été démontré comme pouvant influer sur le risque de transmission verticale (P. ROQUES et al., AIDS, 1993, 7 (suppl. 2), S39-S43 ; F.X. MBOPI KEOU et al., Bull. Inst. Pasteur, 1994, **92**, 3-18; Y.F. KHOURI et al., J. Clin. Invest., 1995, **95**, 732-737 ; G.A. MULDER-KAMPINGA et al., Journal of Virology, 1995, **69**, 2285-2296).

La présente invention s'est, en conséquence, donné pour but de pourvoir à des fragments d'acide nucléique et aux peptides correspondants aptes à servir de réactifs de détection et d'évaluation du risque de transmission materno-foetale du VIH-1.

La présente invention a pour objet un fragment d'acide nucléique, issu du gène codant pour un fragment de la protéine de matrice p17 du VIH-1, essentiellement constitué de 21 à 90 nucléotides et incluant au moins la séquence représentée par la formule Y5 Y6 Y7 Y8 Y9 Y10 Y11, dans laquelle :
Y5 représente AAA, AAG, GAA,
Y6 représente ATA, TTA, CTG, GTA, CTA, GTG, ATG,
Y7 représente GAG, GAA,
Y8 représente GAA, AAG,
Y9 représente ATA, GTA, CTA, GAA, GAG, GAC, GAT, AGA,
Y10 représente CAA, CAG, CGA,
Y11 représente AAA, AGT, AAG, AAT, CAT, AAC, ACC,
à condition que lorsque Y5 représente AAA ou AAG, Y6 soit différent de ATA, Y7 soit différent de GAG ou GAA, Y8 soit différent de GAA, Y9 soit différent de GAG ou GAA, Y10 soit différent de CAA ou CAG et Y11 soit différent de AAT ou AAC.

Une telle séquence code pour un fragment de la protéine de matrice p17 du VIH-1 constitué de 7 à 30 aminoacides et incluant au moins la séquence en aminoacides 103-109 de ladite protéine.

Selon un mode de réalisation avantageux de ladite séquence d'acide nucléique, elle présente la formule suivante :
Y1 GAA Y2 Y3 Y4 Y5 Y6 Y7 Y8 Y9 Y10 Y11 Y12 Y13 Y14 Y15 Y16 Y17
dans laquelle
Y5-Y11 ont la même signification que ci-dessus et
Y1 représente AAA, AAG,
Y2 représente GCT, GCC,
Y3 représente TTA, CTA,
Y4 représente GAT, GAC, GAG,
Y12 représente AGG, AAG, AAA, ATC, AAC,
Y13 représente AGC, AAC, AGT, AAG,
Y14 représente GGG, AAG, AAA, CAG,
Y15 représente CAA, AAA, AGA
Y16 représente AAG, AAA, ACA, CAA, AAC, AAT,
Y17 représente ACA, GCA, AGC, ATA, GCC, GCT, GCG.

De manière préférée, ladite séquence est sélectionnée parmi les séquences suivantes :

- SEQ ID N° 1 (DIA) :
AAA GAA GCT TTA GAT AAA ATA GAG GAA ATA CAA AAA AGG AGC GGG CAA AAG
ACA
- SEQ ID N° 2 (MAM) :
AAA GAA GCC TTA GAC AAA ATA GAG GAA ATA CAA AGT AAG AAC AAG CAA AAG
GCA
- SEQ ID N° 3 (MOU) :
AAG GAA GCT TTA GAT AAA ATA GAG GAA GTA CAG AAA AAG AGC AAG CAA AAG
ACA
- SEQ ID N° 4 (BAB) :
AAG GAA GCC TTA GAT AAA ATA GAG GAA ATA CAA AAG AAG AGC AAG CAA AAG
ACA
- SEQ ID N° 5 (DOS) :
AAG GAA GCT TTA GAT AAA TTA GAG GAA ATA CAA AAT AAG AGC AAA CAA AAG
ACA
- SEQ ID N° 6 (LAN) :
AAG GAA GCT TTA GAT AAG CTG GAG GAA ATA CAA CAT AAG AAC AAG CAA AAG
ACA
- SEQ ID N° 7 (FRA) :
AAG GAA GCC TTA GAT AAA ATA GAG GAG ATA CAA AAT AAG AGC AAG CAA AAA
ACA
- SEQ ID N° 8 (WAN) :
AAG GAA GCT TTA GAT AAA ATA GAG GAA CTA CAA AAG AAG AGT AAG CAA AAA
GCA
- SEQ ID N° 9 (ABD) :
AAG GAA GCT TTA GAT AAG ATA GAG GAA GAA CAA AAC AAA AGT AAG AAA AAA
GCA
- SEQ ID N° 10 (ARI) :
AAG GAA GCT TTA GAT AAG ATA GAG GAA GAG CAA AAC AAA AGT AAG AAA AAA
GCA
- SEQ ID N° 11 (BOI) :
AAG GAA GCT TTA GAG AAG ATA GAG GAA GAG CAA AAC AAA AGT AAG AAA AAG
GCA

- SEQ ID N° 12 (DUM) :
AAG GAA GCT TTA GAG AAG ATA GAG GAA GAG CAA AAC AAA AGT AAG AAA AAA
GCA
- SEQ ID N° 13 (PAL) :
AAG GAA GCT TTA GAT AAG ATA GAG GAA GAG CAA AAC AAA AGT AAG AAA AAA
GCA
- SEQ ID N° 14 (RYO) :
AAG GAA GCT TTA GAC AAG ATA GAA GAA GAG CAA AAC AAA AGT AAG AAA AAA
GCA
- SEQ ID N° 15 (FLO) :
AAG GAA GCT TTA GAG AAG ATA GAG GAA GAG CAA AAC AAA AGT AAG AAA ACA
AGC
- SEQ ID N° 16 (4501) :
AAG GAA GCT TTA GAG AAG ATA GAG GAA GAG CAA AAC AAA AGT AAG AAA CAA
GCA
- SEQ ID N° 17 (FAL) :
AAG GAA GCT TTA GAG AAG ATA GAG GAA GAG CAA AAC AAA AGT AAG AAA AAA
GCA
- SEQ ID N° 18 (HAR) :
AAG GAA GCT CTA GAG AAG GTA GAG GAA GAG CAA AAC AAA AGT AAG AAA AAN
GCA
- SEQ ID N° 19 (RIV) :
AAG GAA GCT TTA GAC AAG ATA GAG GAA GAA CAA AAC AAA AGT AAG AAA AAA
GCA
- SEQ ID N° 20 (CHET) :
AAG GAA GCT TTA GAG AAG ATA GAG GAA GAG CAA AAC AAA AGT AAG AAA AAG
GCA
- SEQ ID N° 21 (CEL) :
AAG GAA GCT TTA GAG AAG GTA GAG GAA GAG CAA ACC AAA AGT AAG AGA AAA
GCA
- SEQ ID N° 22 (SIW) :
AAG GAA GCT CTA GAC AAG ATA GAG GAA GAG CAA AAC AAA AGT AAG AAA AAG
GCA
- SEQ ID N° 23 (MOE) :
AAG GAA GCT TTA GAC AAG ATA GAG GAA GAG CAA ACC AAA AGT AAG AAA AAA
GCA

- SEQ ID N° 24 (GOB) :
AAG GAA GCT TTA GAG AAG ATA GAG GAA GAN CAA ACC AAA AGT AAG AAA AAA
GCA
- SEQ ID N° 25 (4541) :
AAG GAA GCT TTA GAG AAA GTA GAG GAA GAG CAA AAC AAA AGT AAG AAA AAG
GCA
- SEQ ID N° 26 (2754) :
AAG GAA GCT TTA GAG AAG GTA GAG GAA GAG CAA AAC AAA AGT AAG AAA AAA
GCA
- SEQ ID N° 27 (2826) :
AAG GAA GCT TTA GAG AAG ATA GAG GAA GAG CAA AAC AAG AGT AAG AAA AAA
GCA
- SEQ ID N° 28 (MPA) :
AAA GAA GCT CTA GAG GAA GTG GAA AAG AGA CAA AAG ATC AGT CAG CAA AAA
ATA
- SEQ ID N° 29 (IGN) :
AAA GAA GCT CTA GAG GAA GTG GAA AAG AGA CAA AAG ATC AGT CAG AAA AAA
ATA
- SEQ ID N° 30 (OSA) :
AAA GAA GCT CTA GAG GAA GTG GAA AAG GTA CGA AAA AAC AAG CAG CAA AAA
GCA
- SEQ ID N° 31 (BOU):
AAG GAA GCT TTA GAG AAG ATG GAG GAA GAG CAA AAC AAA AGT AAG AAA AAA
GCA ou un fragment de celles-ci, tel que :
SEQ ID N° 32 (sonde 1) : TTTTGTTTTGCTCTTCCTCTATCTT,
SEQ ID N° 33 (sonde 2) : GTTTTGCTCTTCCTCTACC,
SEQ ID N° 34 (sonde 3) : GGTTTGCTCTTCCTCTATC,
SEQ ID N° 35 (sonde 4) : GTTTGCTCTTCCTCTACCT,
SEQ ID N° 56 (sonde 5) : TTTTGTTTTGCTCTTCCTCTAC TTT,
SEQ ID N° 57 (sonde 6) : CTTTTGTTTTGCTCTTCTTCTA TCT.

On entend par séquence d'acide nucléique aussi bien les séquences telles que définies ci-dessus que leurs séquences complémentaires.

La présente invention a également pour objet les séquences peptidiques correspondantes, c'est-à-dire les séquences peptidiques constituées de 7 à 30 aminoacides et incluant au moins la séquence en aminoacides 103 à 109 de la protéine de matrice virale p17, représentée par la formule X2 X3 Glu X4 X5 X6 X7, dans laquelle :
X2 représente Lys, Glu
X3 représente Ile, Leu, Val, Met
X4 représente Glu, Lys
X5 représente Ile, Val, Glu, Arg, Leu
X6 représente Gln, Arg et
X7 représente Lys, Ser, Asn, His, Thr,
à condition que lorsque X2 représente Lys, X3 soit différent de Ile, X4 et X5 soient différents de Glu, X6 soit différent de Gln et X7 soit différent de Asn.

Selon un mode de réalisation avantageux de ladite séquence, elle présente la formule suivante :
Lys Glu Ala Leu X1 X2 X3 Glu X4 X5 X6 X7 X8 X9 X10 X11 X12 X13,
dans laquelle
X2-X7 ont la même signification que ci-dessus et
X1 représente Asp, Glu
X8 représente Arg, Lys, Ile, Asn
X9 représente Ser, Asn, Lys
X10 représente Gly, Lys, Gln, Glu
X11 représente Gln, Lys, Arg
X12 représente Lys, Gln
X13 représente Thr, Ala, Ile.

De manière préférée, ladite séquence est sélectionnée parmi les séquences suivantes :

- SEQ ID N° 36 (DIA) :
Lys Glu Ala Leu Asp Lys Ile Glu Glu Ile Gln Lys Arg Ser Gly Gln Lys Thr
- SEQ ID N° 37 (MAM) :
Lys Glu Ala Leu Asp Lys Ile Glu Glu Ile Gln Ser Lys Asn Lys Gln Lys Ala
- SEQ ID N° 38 (MOU) :
Lys Glu Ala Leu Asp Lys Ile Glu Glu Val Gln Lys Lys Ser Lys Gln Lys Thr
- SEQ ID N° 39 (BAB) :
Lys Glu Ala Leu Asp Lys Ile Glu Glu Ile Gln Lys Lys Ser Lys Gln Lys Thr

- SEQ ID N° 40 (DOS) :
Lys Glu Ala Leu Asp Lys Leu Glu Glu Ile Gln Asn Lys Ser Lys Gln Lys Thr
- SEQ ID N° 41 (LAN) :
Lys Glu Ala Leu Asp Lys Leu Glu Glu Ile Gln His Lys Asn Lys Gln Lys Thr
- SEQ ID N° 42 (FRA) :
Lys Glu Ala Leu Asp Lys Ile Glu Glu Ile Gln Asn Lys Ser Lys Gln Lys Thr
- SEQ ID N° 43 (WAN) :
Lys Glu Ala Leu Asp Lys Ile Glu Glu Leu Gln Lys Lys Ser Lys Gln Lys Ala
- SEQ ID N° 44 (ABD) :
Lys Glu Ala Leu Asp Lys Ile Glu Glu Glu Gln Asn Lys Ser Lys Lys Lys Ala
- SEQ ID N° 45 (BOI) :
Lys Glu Ala Leu Glu Lys Ile Glu Glu Glu Gln Asn Lys Ser Lys Lys Lys Ala
- SEQ ID N° 46 (4501) :
Lys Glu Ala Leu Glu Lys Ile Glu Glu Glu Gln Asn Lys Ser Lys Lys Gln Ala
- SEQ ID N° 47 (HAR) :
Lys Glu Ala Leu Glu Lys Val Glu Glu Glu Gln Asn Lys Ser Lys Lys Lys Ala
- SEQ ID N° 48 (CEL) :
Lys Glu Ala Leu Glu Lys Val Glu Glu Glu Gln Thr Lys Ser Lys Arg Lys Ala
- SEQ ID N° 49 (SIW)
Lys Glu Ala Leu Glu Lys Ile Glu Glu Glu Gln Asn Lys Ser Glu Lys Lys Ala
- SEQ ID N° 50 (MOE) :
Lys Glu Ala Leu Asp Lys Ile Glu Glu Glu Gln Thr Lys Ser Lys Lys Lys Ala
- SEQ ID N° 51 (GOB) :
Lys Glu Ala Leu Glu Lys Ile Glu Glu Glu Gln Thr Lys Ser Lys Lys Lys Ala
- SEQ ID N° 52 (MPA) :
Lys Glu Ala Leu Glu Glu Val Glu Lys Arg Gln Lys Ile Ser Gln Gln Lys Ile
- SEQ ID N° 53 (IGN) :
Lys Glu Ala Leu Glu Glu Val Glu Lys Arg Gln Lys Lys Ser Lys Gln Lys Ile
- SEQ ID N° 54 (OSA) :
Lys Glu Ala Leu Glu Glu Val Glu Lys Val Arg Lys Asn Lys Gln Gln Lys Ala
- SEQ ID N° 55 (BOU) :
Lys Glu Ala Leu Glu Lys Met Glu Glu Glu Gln Asn Lys Ser Lys Lys Lys Ala

De manière avantageuse, lesdits peptides peuvent être préparés par synthèse, selon des méthodes connues.

De manière inattendue, la Demanderesse a trouvé que, selon le sous-type considéré, ces fragments d'acide nucléique et les peptides qui leur correspondent sont significativement associés à la fréquence de transmission materno-foetale du VIH-1.

En particulier, les séquences suivantes (nucléiques et peptidiques) sont considérées comme transmissibles :

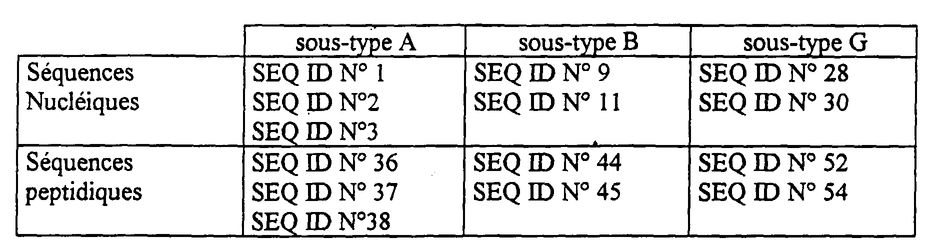

De telles données démontrent pour la première fois, l'association entre des marqueurs moléculaires viraux et la présence d'un risque élevé ou faible de transmission materno-foetale du VIH-1.

La présente invention a également pour objet des anticorps anti-peptides tels que définis ci-dessus.

Selon un mode de réalisation avantageux desdits anticorps, ils consistent en anticorps polyclonaux.

Selon un autre mode de réalisation avantageux desdits anticorps, ils consistent en anticorps monoclonaux.

Les anticorps polyclonaux sont avantageusement obtenus par immunisation d'un animal (mammifère ou gallinacé notamment, etc...) approprié avec un peptide selon l'invention, éventuellement couplé à une protéine convenable telle que la sérum albumine bovine (SAB) ou la KLH.

Les anticorps monoclonaux anti-fragments peptidiques sont avantageusement obtenus de manière connue en soi, par fusion de cellules spléniques de souris immunisées avec un antigène consistant en un fragment peptidique tel que défini ci-dessus, éventuellement couplé à une protéine convenable telle que la SAB ou la KLH, avec des cellules myélomateuses appropriées.

La présente invention a également pour objet un réactif pour le dépistage et l'évaluation du risque de transmission materno-foetale du VIH-1, caractérisé en ce qu'il est sélectionné dans le groupe constitué par les séquences nucléotidiques, les fragments peptidiques et les anticorps anti-peptides selon l'invention.

En particulier, ledit réactif est avantageusement constitué soit d'un ensemble de peptides, soit d'un ensemble d'anticorps anti-peptidiques selon l'invention, soit d'un ensemble de séquences nucléotidiques, tels que définis ci-dessus.

Selon un mode de réalisation avantageux de l'invention, lorsque ledit réactif est constitué d'au moins une séquence d'acide nucléique, il comprend au moins une séquence codant pour les amino-acides 103-109 de la protéine p17 du VIH-1, laquelle séquence nucléique est représentée par la formule Y5 Y6 Y7 Y8 Y9 Y10 Y11, dans laquelle :
Y5 représente AAA, AAG, GAA,
Y6 représente ATA, TTA, CTG, GTA, CTA, GTG, ATG,
Y7 représente GAG, GAA,
Y8 représente GAA, AAG,
Y9 représente ATA, GTA, CTA, GAA, GAG, GAC, GAT, AGA,
Y10 représente CAA, CAG, CGA,
Y11 représente AAA, AGT, AAG, AAT, CAT, AAC ou
il est apte à détecter spécifiquement une séquence nucléique contenant ladite formule.

Conformément à ce mode de réalisation, ledit réactif est notamment sélectionné dans le groupe constitué par les séquences nucléotidiques ou les fragments de celles-ci, telles que définies ci-dessus, associées à au moins un moyen de détection approprié.

Parmi les fragments de séquences nucléiques particulièrement intéressants, on peut citer : 
- sonde 1 (SEQ ID N° 32) : TTTTGTTTTGCTCTTCCTCTATCTT,
- sonde 2 (SEQ ID N° 33) : GTTTTGCTCTTCCTCTACC,
- sonde 3 (SEQ ID N° 34) : GGTTTGCTCTTCCTCTATC,
- sonde 4 (SEQ ID N° 35) : GTTTGCTCTTCCTCTACCT,
- sonde 5 (SEQ ID N° 56) : TTTTGTTTTGCTCTTCCTCTAC
TTT,
 
- sonde 6 (SEQ ID N° 57) : CTTTTGTTTTGCTCTTCTTCTA
TCT,
 particulièrement spécifiques pour dépister et évaluer le risque de transmission du VIH-1 dans le sous-type B.

La sonde 1 permet de détecter au moins les séquences ARI (SEQ ID N° 10), BAR et BOI (SEQ ID N° 11), la sonde 2 permet de détecter au moins les séquences HAR (SEQ ID N° 18), LOUB, VIL, et 2754 (SEQ ID N° 26), la sonde 3 permet de détecter au moins les séquences FAL (SEQ ID N° 17), HMI, AMO, CHET (SEQ ID N° 20), GOB (SEQ ID N° 24), MOE (SEQ ID N° 23), SIW (SEQ ID N° 22), FLO (SEQ ID N° 15), 2758, 2826 (SEQ ID N° 27), 4501 (SEQ ID N°16), 4538 et 5613, la sonde 4 permet de détecter au moins la séquence CEL (SEQ ID N° 21), la sonde 5 permet de détecter au moins la séquence 4541 (SEQ ID N° 25) et la sonde 6 permet de détecter au moins la séquence RYO (SEQ ID N° 14).

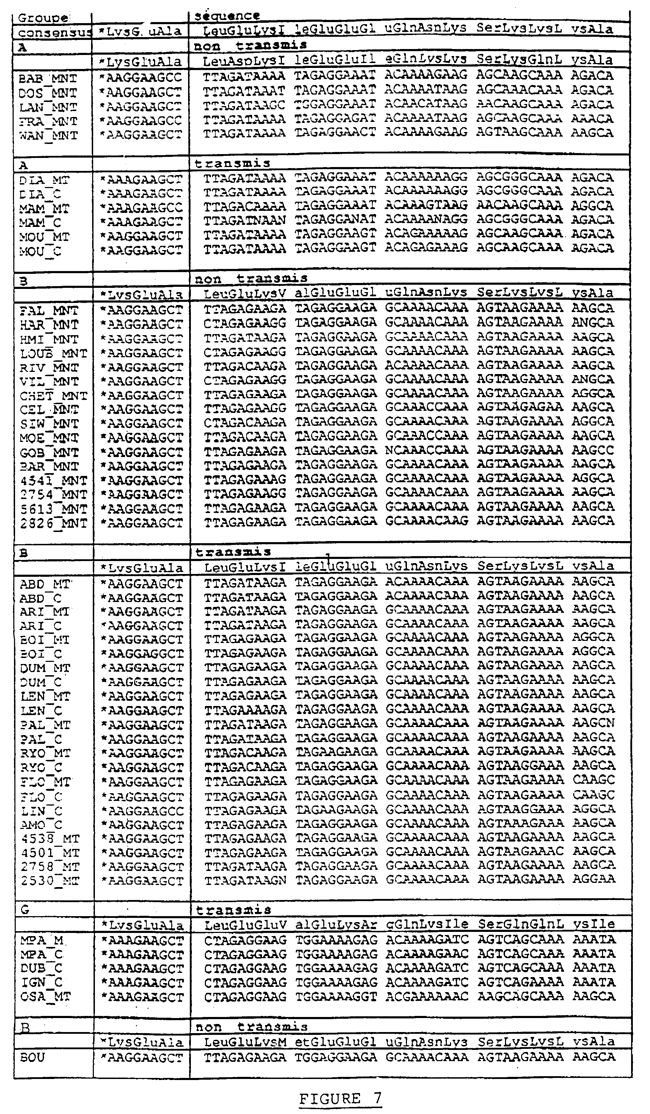

Les autres séquences sont représentées à la figure 7.

Le marqueur est avantageusement choisi dans le groupe qui comprend notamment les isotopes radioactifs, les enzymes appropriées, les fluoro-chromes et les marqueurs chimiques appropriés.

Selon un autre mode de réalisation avantageux de l'invention, lorsque ledit réactif est constitué d'au moins un peptide, il comprend au moins une séquence en amino-acides 103-109 de la protéine p17 du VIH-1, représentée par la formule X2 X3 Glu X4 X5 X6 X7, dans laquelle :
X2 représente Lys, Glu
X3 représente Ile, Leu, Val, Met
X4 représente Glu, Lys
X5 représente Ile, Val, Glu Arg
X6 représente Glu, Arg et
X7 représente Lys, Ser, Asn, His, Thr ou
il est apte à détecter spécifiquement un peptide contenant ladite formule.

La présente invention a également pour objet un procédé de détection, de dépistage et d'évaluation du risque de transmission materno-foetale du VIH-1, dans un échantillon biologique, caractérisé en ce qu'il comprend une étape dans laquelle l'on met en contact l'échantillon biologique avec un réactif conforme à l'invention et une étape dans laquelle on détecte une interaction spécifique entre ledit réactif et une ou plusieurs séquences de l'ADN dudit virus, consistant en l'une des séquences d'acide nucléique telles que définies ci-dessus et présentes dans ledit échantillon biologique.

Selon un mode de mise en oeuvre préféré de ce procédé, la détection de l'une des séquences telles que définies ci-dessus est effectuée après amplification par la méthode d'amplification PCR (amplification en chaîne par la Taq polymérase avec des amorces appropriées) de la région codant pour la protéine de matrice virale p17, suivie de l'hybridation du produit obtenu sur membrane sur laquelle ont préalablement été fixées une ou plusieurs des sondes telles que définies ci-dessus.

La présente invention a, en outre, pour objet un kit, coffret ou ensemble coordonné prêt à l'emploi, pour la mise en oeuvre du procédé de détection, de dépistage et d'évaluation du risque de transmission materno-foetale du VIH-1, conforme à l'invention, caractérisé en ce qu'il comprend outre des quantités de tampons et de réactifs appropriés pour la mise en oeuvre de ladite détection, des doses appropriées d'au moins une sonde ou fragment de sonde nucléotidique conforme à l'invention.

Conformément à l'invention, lorsque ladite sonde est sélectionnée parmi les séquences ID N° 32, 33, 34, 35, 56 ou 57, ledit kit est particulièrement adapté à la détection, au dépistage et à l'évaluation du risque de transmission du VIH-1 de sous-type B.

Selon un mode de réalisation avantageux du kit ou nécessaire conforme à la présente invention, celui-ci comporte les éléments suivants :
- les réactifs nécessaires pour effectuer une amplification PCR d'ADN,
- des doses appropriées d'amorces spécifiques de la région p17 du gène gag du VIH-1, et
- au moins une séquence nucléotidique conforme à l'invention, capable de s'hybrider, dans des conditions stringentes, à la partie de la séquence de la région p17 du gène gag du VIH-1 comprenant l'une des séquences nucléotidiques telles que définies ci-dessus.

Des conditions stringentes, au sens de la présente invention, sont notamment les suivantes : hybridation en présence de SSPE 5X, 0,5 % SDS pendant 30 min à des températures, qui dépendent des Tm de chaque oligosonde préfixée (45 à 60°C).

La présente invention a également pour objet un procédé de détection, de dépistage et d'évaluation du risque de transmission materno-foetale du VIH-1, caractérisé en ce qu'il consiste à détecter l'une des séquences peptidiques telles que définies ci-dessus dans un échantillon biologique maternel, en mettant en présence ledit échantillon biologique avec un pool d'anticorps spécifiques desdites différentes séquences peptidiques.

La présente invention a également pour objet un procédé de détection, de dépistage et d'évaluation du risque de transmission materno-foetale du VIH-1, caractérisé en ce qu'il consiste à détecter l'un des anticorps anti-peptides tels que définis ci-dessus, dans un échantillon biologique maternel, en mettant en présence ledit échantillon biologique avec un *pool* de peptides conformes à l'invention.

Selon un mode de mise en oeuvre particulièrement avantageux de ce procédé, ledit *pool* de peptides est constitué des séquences ID N° 44, 45, 47, 48, 50 et 51.

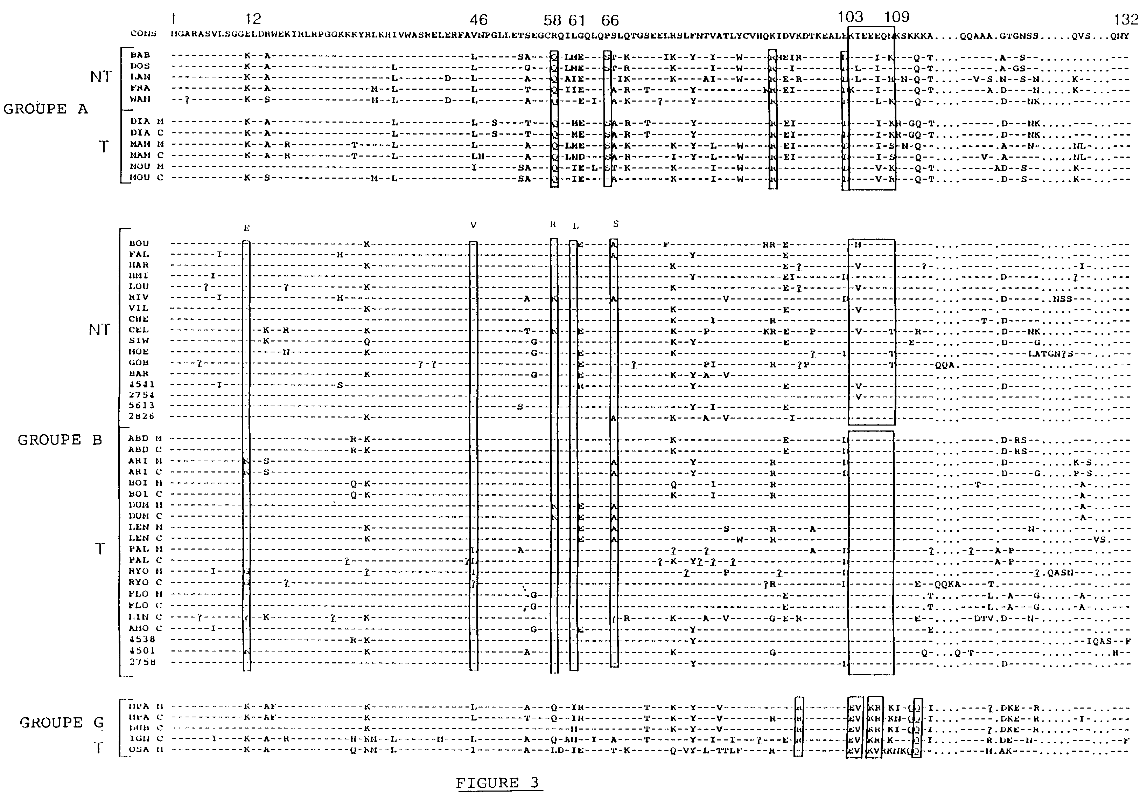

De tels réactifs et procédés de détection et d'évaluation selon l'invention permettent effectivement d'évaluer le risque de transmission materno-foetale, dans la mesure où les virus contenant certaines des séquences précitées ne sont jamais transmis à l'enfant pendant la grossesse, alors que d'autres le sont systématiquement et notamment les SEQ ID N° 44 et SEQ ID N° 45 (voir également figure 3).

La présente invention a, en outre, pour objet un kit, coffret ou ensemble coordonné prêt à l'emploi, pour la mise en oeuvre du procédé de détection, de dépistage et d'évaluation du risque de transmission materno-foetale du VIH-1, conforme à l'invention, caractérisé en ce qu'il comprend outre des quantités de tampons et de réactifs appropriés pour la mise en oeuvre de ladite détection, un support solide approprié convenablement revêtu d'au moins un ligand choisi dans le groupe qui comprend les peptides conformes à l'invention et les anticorps conformes à l'invention, au moins un flacon contenant des conjugués choisis dans le groupe qui comprend les conjugués enzyme appropriée-anticorps anti-Ig humains appropriés, de chèvre notamment, et les conjugués enzyme appropriée-anticorps anti-peptides conformes à l'invention et des quantités ou doses appropriées d'une substance de révélation appropriée.

Outre les dispositions qui précédent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- les figures 1A et 1B représentent les produits obtenues par PCR ; figure 1A : gel d'agarose : les produits obtenus après amplification par PCR du gène codant pour la protéine p17 ; figure 1B: localisation des amorces utilisées dans les PCR emboîtées,
- la figure 2 représente l'arbre phylogénique consensus des séquences nucléotidiques,
- la figure 3 représente l'alignement multiple des peptides issus de la protéine p17 de VIH-1 de différents isolats viraux et leur corrélation avec la transmission materno-foetale,
- les figures 4A et 4B représentent la relation entre la séquence peptidique 103-109 et la transmission verticale du VIH-1 dans le sous-type B,
- la figure 5 illustre les différences génotypiques entre les sous-types A, B, G et D,
- la figure 6 illustre les résultats obtenus lors de l'évaluation du risque de transmission materno-foetale du VIH-1 de sous-type B,
- la figure 7 représente l'alignement multiple des séquences nucléotidiques codant pour la protéine p17 de VIH-1 de différents isolats viraux et leur corrélation avec la transmission materno-foetale.

Il doit être bien entendu, toutefois, que ces exemples sont donnés uniquement à titre d'illustration de l'objet de l'invention, dont ils ne constituent en aucune manière une limitation.

### EXEMPLE 1 : Détection rapide d'isolats maternels du VIH-1 à fort ou faible risque de transmission verticale par la technique de reverse dot blot appliquée au gêne de la p17 (protéine de matrice) (technique mettant en oeuvre une seule PCR) ; application au sous-type B.

### 1) Préparation d'ADN cellulaires totaux :

- matériel : 5 à 10 ml de sang total prélevé sur anticoagulant (héparine ou citrate),
- séparation des cellules mononucléées du sang périphérique total des patients sur gradient de Ficoll,
- lyse des cellules en tampon Tris HCl 10 mM pH 7,8, EDTA 1 mM, SDS 0,5 % additionné de 20 mg/ml de protéinase K 2 heures à 56°C,
- extraction des acides nucléiques par phénol, chloroforme et alcool isoamylique,
- précipitation en acétate de sodium 3 M pH 5,4 et isopropanol (1 vol/10 vol),
- lavage en éthanol 70 % et mise en solution en TE (Tris HCI 10 mM pH 7,5, EDTA 0,1 mM).

### 2) Amplification par PCR du gène de la p17:

- amorce sens : U5Rnp ^{5'}ACTCTGGTAGCTAGAGATCCCT^{3'} biotinylée en 5',
- amorce antisens: 781cp 5'-GGTGATATGGCCTGATGTACCAT-3' (781-759).
- protocole opératoire :
   pour 100 µl de volume réactionnel final : tampon PCR 10X (Appligène) à 1,5 mM MgCl₂, 200 µM des désoxynucléotides, 0,25 µM de chaque amorce, 0,6 U Taq polymérase (Appligène)/ml.

une étape de 3 min à 94°C : 38 cycles de 1 min à 57°C, 1 min à 72°C, 45 sec à 94°C ; une étape de 2 min à 57°C et 10 min à 72°C.

### 3) Préparation des sondes oligonucléotidiques:

### 6 sondes nucléotidiques de séquences respectives (5'-3'):

sonde 1 (SEQ ID N° 32) : TTTTGTTTTGCTCTTCCTCTATCTT,

sonde 2 (SEQ ID N° 33) : GTTTTGCTCTTCCTCTACC,
sonde 3 (SEQ ID N° 34) : GGTTTGCTCTTCCTCTATC,
sonde 4 (SEQ ID N° 35) : GTTTGCTCTTCCTCTACCT,
sonde 5 (SEQ ID N° 56) : TTTTGTTTTGCTCTTCCTCTAC TTT[?]
sonde 6 (SEQ ID N° 57) : CTTTTGTTTTGCTCTTCTTCTA TCT[?]

De manière surprenante, les sondes ID N° 32 et ID N° 57 permettent de détecter des VIH-1 toujours transmis au foetus, alors que les sondes 33, 34, 35 et 56 permettent de détecter des VIH-1 qui ne sont jamais transmis au foetus.

Addition d'une queue Poly(dT) en 3' de chaque sonde oligonucléotidique :
200 pmol de chaque sonde plus 80 nmol de dTTP dans 100 µl de tampon (100 mM de cacodylate de potassium, Tris-HCl 25 mM, CoCl₂ 1 mM, DTT 0,2 mM pH 7,6) avec 60 unités (50 pmol) de terminale déoxyribonucléotidyltransférase (Boerhinger). 60 min à 37°C puis arrêt par addition de 100 µl d'EDTA 10 mM.

### 4) Fixation des sondes sur membrane :

Dilution des sondes dans 100 µl de TE (Tris-HCl 10 mM, EDTA 1 mM, pH 8) puis fixation sur membrane de nylon (Genetran-45) à l'aide d'un appareil pour dot-blot (Bio-Dot, Biorad) puis fixation aux UV dans un four Spectrolinker (Spectronics Corporation), à une énergie de 120 mJ/unité de surface.

Lavage des membranes dans 200 ml de SSPE 5X (SSPE 1X = 180 mM Nacl, 10 mM NaH₂PO₄, EDTA 1 mM, pH 7,2) avec 0,5 % de SDS pendant 30 min à 55°C.

### 5) Hybridation des produits de PCR sur membrane :

Dénaturation des produits de PCR, 3 min à 90°C, puis hybridation en parallèle avec des témoins PCR de séquences connues :
- hybridation sur membrane en SSPE 5X 0,5 % SDS pendant 30 min à des températures fonctions des Tm de chaque oligosonde préfixée (45 à 60°C),
- lavage en SSPE 2X 0,1 % SDS entre 45 et 60°C.

### 6) Révélation par addition de peroxydase de raifort HRP couplée à la streptavidine puis d'un substrat chimioluminescent:

- incubation des membranes dans 1 à 2 ml de SSPE 2X 0,1 % SDS contenant 10 à 20 ml de HRP-streptavidine (5 mg/ml, Perkin-Elmer Cetus) pendant 15 min, puis lavage 10 min dans le même tampon,
- détection par substrat chimioluminescent.

### 7) Interprétation des résultats:

Si l'on obtient une hybridation seulement avec les sondes 1 ou 6 (sonde 1 ou 6 positives) et quels que soient les résultats obtenus avec les sondes 2, 3, 4 et 5, il existe un risque important de transmission mère-enfant.

S'il n'y a pas d'hybridation en présence des sondes 1 et 6 (sondes 1 et 6 négatives), et si les résultats obtenus avec la(les) sonde(s) 2 et/ou 4 et/ou 5 sont positifs, le risque est faible.

Si les sondes 1, 2, 4 et 5 sont négatives (absence d'hybridation) et la sonde 3 positive, le risque est probablement faible.

Si toutes les sondes sont négatives (absence d'hybridation), il conviendra de tester un nouveau prélèvement. En cas de confirmation, l'isolat viral maternel appartient à un autre sous-type du VIH-1 que le sous-type B.

### EXEMPLE 2 : Détection rapide d'isolats maternels du VIH-1 à fort ou faible risque de transmission verticale par la technique de reverse dot blot appliquée au gêne de la p17 (protéine de matrice) (technique mettant en oeuvre deux PCR successives :PCR emboîtées) ; application au sous-type B.

### 1) Préparation d'ADN cellulaires totaux:

On procède comme à l'exemple 1.

### 2) Amplification par PCRs emboîtées du gène de la p17:

- PCR primaire : amorces externes

amorce sens : U5Rnp 5'-ACTCTGGTAGCTAGAGATCCCT-3'
amorce anti-sens : SK39 5'-TTTGGTCCTTGTCTTATGTCCA
GAATGC-3'

Ces amorces correspondent respectivement aux nucléotides 1-18 et 1204-1177 du génome de la souche VIH-1 LAI.
- PCR secondaire : amorces internes

amorce sens : U5Rnp 5'-ACTCTGGTAGCTAGAGATCCCT-3'
biotinylée en 5' (BioU5Rnp),
amorce antisens : 781cp 5'-GGTGATATGGCCTGATGTACCAT-
3' (781-759).

Le protocole opératoire est identique à celui décrit à l'exemple 1.

La PCR secondaire est réalisée à partir de 2 µl de la PCR primaire.

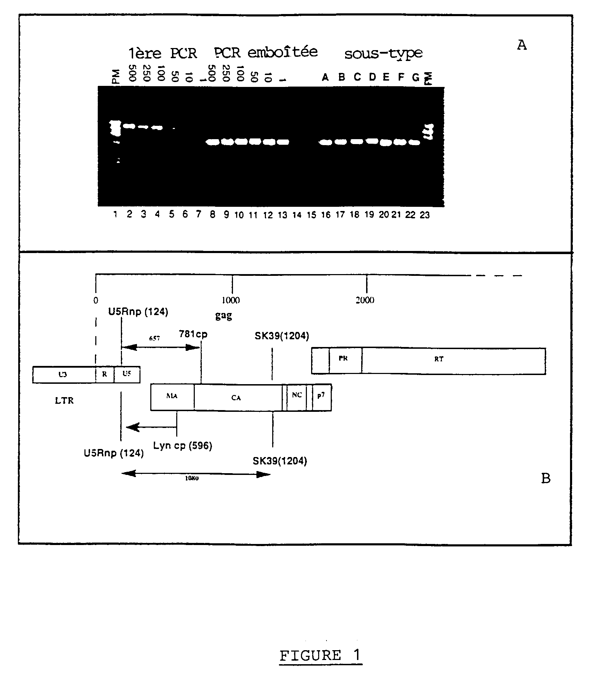

Pour identifier les produits amplifiés par lesdites PCR, on dépose 5 µl de chaque échantillon sur un gel d'agarose à 1 %, on procède à une migration électro-phorétique et on révèle par coloration au bromure d'éthidium. Les résultats sont illustrés à la figure 1A, dans laquelle la piste 1 correspond au marqueur de poids moléculaire (digestion de φX174 par l'enzyme Hind III); les pistes 2 à 7 correspondent respectivement à 500, 250, 100, 50, 10 et 1 copies de l'ADN de provirus intégré extrait de cellules 85-14 F2, amplifié avec la paire d'amorces U5Rnp-SK39; les pistes 8 à 13 correspondent aux produits de la deuxième PCR réalisée avec la paire d'amorces BioU5Rnp-781cp, à partir du produit obtenu avec la première PCR (pistes 2 à 7) ; les pistes 16 à 22 correspondent aux produits d'amplification PCR d'ADN viral respectivement des sous-types A, B, C, D, E, F et G, obtenus à l'aide de la paire d'amorces BioU5Rnp-781cp (A = isolat BAB-MNT; B = isolat FAL-MNT; C = souche 15166; D = souche NDK; E = souche Viet 26 ; F = souche 1011; G = isolat MPA-M).

Cette figure montre que les limites de détection des PCR externe et interne, déterminées en utilisant les cellules standard 85-14 F2 comme précisé ci-dessus sont respectivement 10 et 1 copies.

La figure 1B montre la position des paires d'amorces utilisées sur le génome de la souche VIH-1 LAI.

Cet ensemble d'amorces est particulièrement intéressant pour amplifier tous les isolats issus des sous-types de VIH-1 A, B, C, D, E, F et G (figure 1A).

Parallèlement, pour la détection des isolats dans les échantillons maternels et foetaux, on procède comme précisé aux étapes 3 à 7 de l'exemple 1.

L'interprétation des résultats est identiques à celle de l'exemple 1; toutefois, l'utilisation de 2 PCR emboîtées rend la méthode plus sensible.

### EXEMPLE 3 : Détection rapide des mères infectées par VIH-1 ayant un fort risque de transmission verticale, par la technique d'ELISA. (Détail du protocole opératoire pour le sous-type B du VIH-1.)

### 1) Prélèvement sérique.

- matériel: 2 à 5 ml de sang total prélevé sur tube sec
- séparation du caillot sanguin par centrifugation
- stockage des sérums en deux aliquots dans des tubes à congélation à -20°C.

### 2) Préparation des plaques ELISA et des témoins positifs.

### 2-1) synthèse chimique des peptides cibles

6 peptides de séquences respectives (N -vers C) :

1 2 3 4 5 6 7 8 9 10 11 12 13 14 15 16 17 18
peptide 1 (SEQ ID N° 45) :
Lys Glu Ala Leu Glu Lys Ile Glu Glu Glu Gln Asn Lys Ser Lys Lys Lys Ala
peptide 2 (SEQ ID N° 44) :
Lys Glu Ala Leu Asp Lys Ile Glu Glu Glu Gln Asn Lys Ser Lys Lys Lys Ala
peptide 3 (SEQ ID N° 47) :
Lys Glu Ala Leu Glu Lys Val Glu Glu Glu Gln Asn Lys Ser Lys Lys Lys Ala
peptide 4 (SEQ ID N° 51) :
Lys Glu Ala Leu Glu Lys Ile Glu Glu Glu Gln Thr Lys Ser Lys Lys Lys Ala
peptide 5 (SEQ ID N° 48) :
Lys Glu Ala Leu Glu Lys Val Glu Glu Glu Gln Thr Lys Ser Lys Arg Lys Ala
peptide 6 (SEQ ID N° 50) :
Lys Glu Ala Leu Asp Lys Ile Glu Glu Glu Gln Thr Lys Ser Lys Lys Lys Ala

Un peptide non-spécifique de HIV SP951481 est utilisé comme témoin négatif (P-).

### 2-2 ) préparation d'un pool d'anticorps témoins :

Le *pool* d'anticorps utilisé est un *pool* de sérum de patients séropositifs.

### 2-3) fixation des peptides sur barette de 8 puits.

### 2-3-1) matériel : Barette 8 puits (Costar Labcoat Amine Binding ref 2506)

plaque Immulon 4 de Dynatech réf. 15415,
peptide synthétisé tel que décrit plus haut,
peptide non-spécifique de HIV SP951481,
un *pool* d'antigènes viraux servant de témoins positifs de la réponse anti-HIV des sérums : lysat de cellules chroniquement infectées par HTLVIII (50 millions de cellules H9V3 lysées par 2 ml d'une solution à 1 % de détergent NP40),
lait écrémé Gloria®,
ortho-phénylène-diamine en tablette de 30 mg (OPD, Sigma chimie, France),
incubateur à 37°C,
pipette multi-distributrice.

### 2-3-2) protocole :

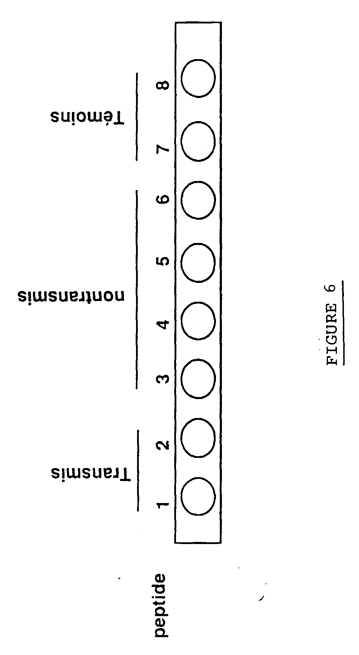

- mise en solution des peptides à la concentration de 2 mg/ml dans un tampon carbonate pH 9,6 (NaHCO₃ 50 mM ; NaCl 0,15 M). Les peptides sont dilués avant emploi à une concentration de 2 µg/ml.
- on distribue 100 µl de solution dans chaque puits de la façon suivante : les peptides 1 à 6 dans les puits 1 à 6 respectivement ; dans le puits 7, on introduit un peptide non-spécifique de HIV (SP951481) (P-) ; enfin dans le puits 8, le lysat de H9V3 (témoin positif de la réponse anti-HIV) dilué au 1/100 dans un tampon PBS (phosphate 0,1 M, pH 7,5, 0,15 M NaCl) (voir figure 6).
- les plaques (chacune suffisante pour tester 10 sérums) sont incubées 2 heures à 37°C.
- on élimine le réactif, on lave deux fois avec 200 µl par puits d'une solution de PBS/Tween 20 à 0,05 % (tampon A).
- on sature les plaques (blocage de la fixation non-spécifique des antigènes dans les puits), en distribuant 300 µl d'une solution de lait écrémé à 3 % dans le PBS/0,05 % Tween 20, suivi d'une incubation de 2 heures à 37°C.
- on lave à nouveau les plaques, comme précédemment.

Les plaques ou les barettes peuvent être stockées après dessiccation, à 4°C.

### 3) ELISA

### 3-1) protocole

- Les sérums à tester, inactivés à 56°C 35 min. sont dilués au 1/100 et au 1/1000 dans du Tampon B : PBS-Tween® 20 0,05 % ; lait écrémé 3 % (Gloria, Nestlé).
- Le *pool* d'anticorps préparés comme décrit en 2-2 est dilué au 1 000ème dans le tampon B : solution C.
- 100 µl de chaque sérum sont distribués dans les puits correspondants aux différents antigènes.
- L'absorption se fait en 1 heure à 37°C en atmosphère humide.
- Les puits sont lavés 3 fois avec le tampon A.
- Les puits sont saturés avec 300 µl de la solution C, incubés 1 h à 37°C, lavés 3 fois avec PBS Tween® 20 0,1%.
- On ajoute 100 µl de sérum de chèvre anti-IgG humaines (Cappel Organon Teknika Cat. Réf. 3201-0231), couplé à la peroxydase, dilué au 1/10000 dans le tampon B.
- 5 lavages sont effectués: 4 en PBS-Tween 20 0,1% et 1 en PBS seul.

### 3-2) révélation

- dans chaque puits on ajoute 100 µl d'une solution D :
   0,05 M tampon citrate pH 5,6, et 0,01% H₂O_{2,} 3 mg/ml d'ortho-phénylène-Diamine (OPD, Sigma chimie, France).
- on incube 30 min à température ambiante, à l'obscurité.
- on arrête la réaction avec 50 µl d'acide sulfurique 4N.
- la lecture de la densité optique à 492 et 630 nm est effectuée par un lecteur de plaque (automated microplate reader EL311, Bio-Tek Instrument inc. USA).

### 3-3) Analyse des résultats :

Une densité optique supérieure à 0,1 est considérée comme constituant un signal positif (détection du peptide correspondant).

La détermination de la présence d'anticorps contre le virus susceptible d'être transmis correspondra à un signal positif dans les puits 1 ou 2 (voir figure 6).

En cas de signal positif dans les puits 1 ou 2 et dans un quelconque des puits 3 à 4 : la mère présente toujours un risque de transmission plus élevé.

Le faible risque de transmission sera validé si et seulement si il y a une réponse positive du sérum de la mère contre l'un quelconque des peptides 3 à 6 (signal positif dans l'un des puits 3 à 6) et pas de signal positif dans les puits 1, 2 et 7 ou 8 (peptides 1, 2 et témoins).

Le risque sera considéré non évaluable si et seulement si il n'y a pas de signal positif avec l'un quelconque des peptides 1 à 6 dans un essai effectué avec au moins deux dilutions du sérum de la mère.

En effet, il existe quelques cas de femmes ne présentant pas d'anticorps anti-p17 et la disparition de ces anticorps a été corrélée avec l'avancement dans la maladie. Donc, une réponse positive dans les puits 3 à 6 sera considérée comme un bon témoin de la validité du test.

Le Tableau I ci-après illustre les mêmes résultats de manière quantitative.

Ces résultats sont exprimés en densité optique des dilutions au 1/100ème de différents sérums : sérums témoins positifs (T+) *(pool* de sérums de patients), sérums témoins négatifs (T-) et sérums à tester, en présence des peptides 1 à 6, du peptide contrôle (P-) et d'un lysat de virus.

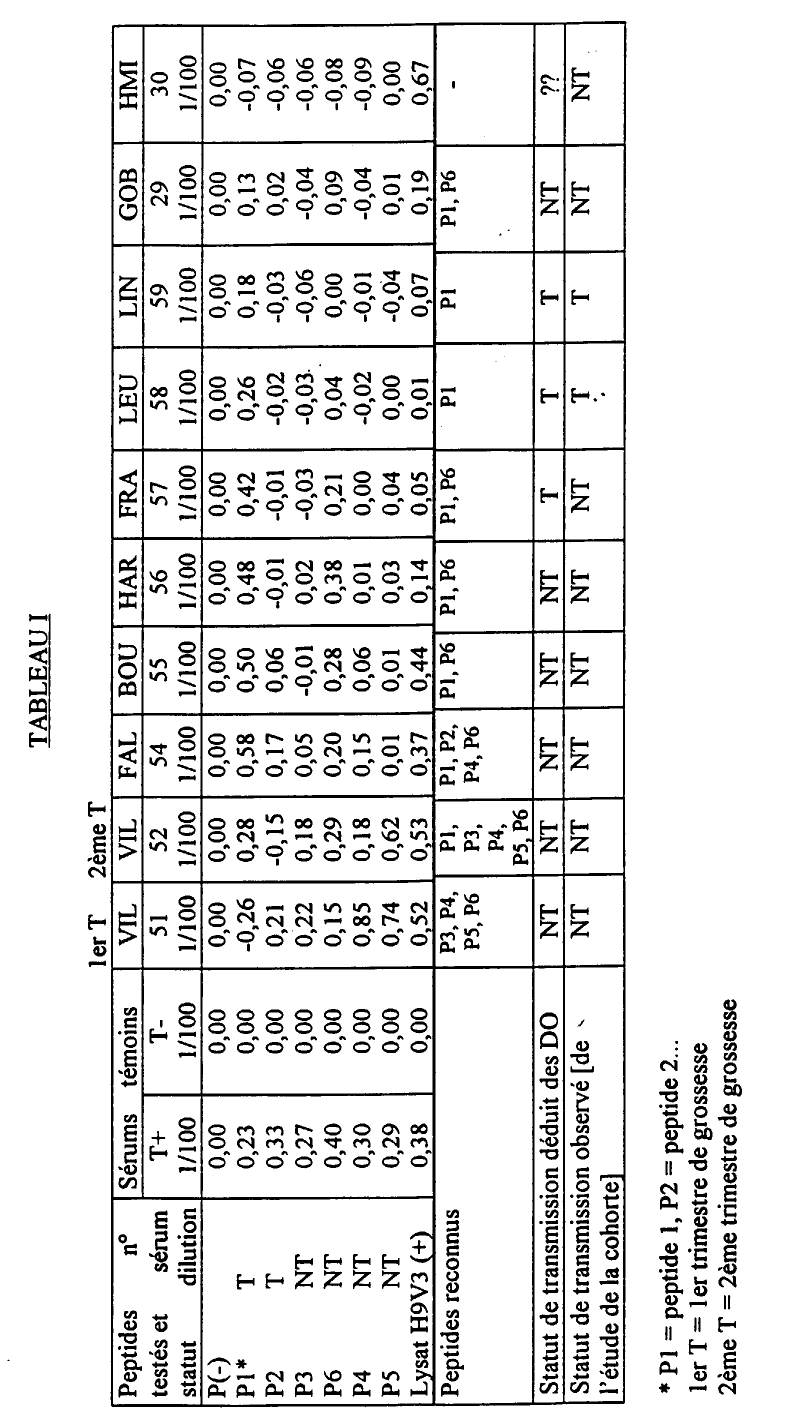

### 4) Mise en évidence de la corrélation de la transmission materno-foetale du VIH avec les séquences décrites précédemment et identifiées dans les exemples 1 et 2:

Les précisions suivantes ont été apportées par le séquençage de virus, obtenus d'une cohorte contrôle :

Des séquences VIH isolées de différentes mères et enfants ont été séquencées par la méthode de séquençage direct en phase solide, à partir de 12 paires mères infectées par VIH-1 enfant, 4 enfants, 4 mères transmettrices et 22 mères non transmettrices.

Les échantillons sanguins sont collectés au moment de l'accouchement pour les mères et pendant le premier mois de la vie pour les enfants.

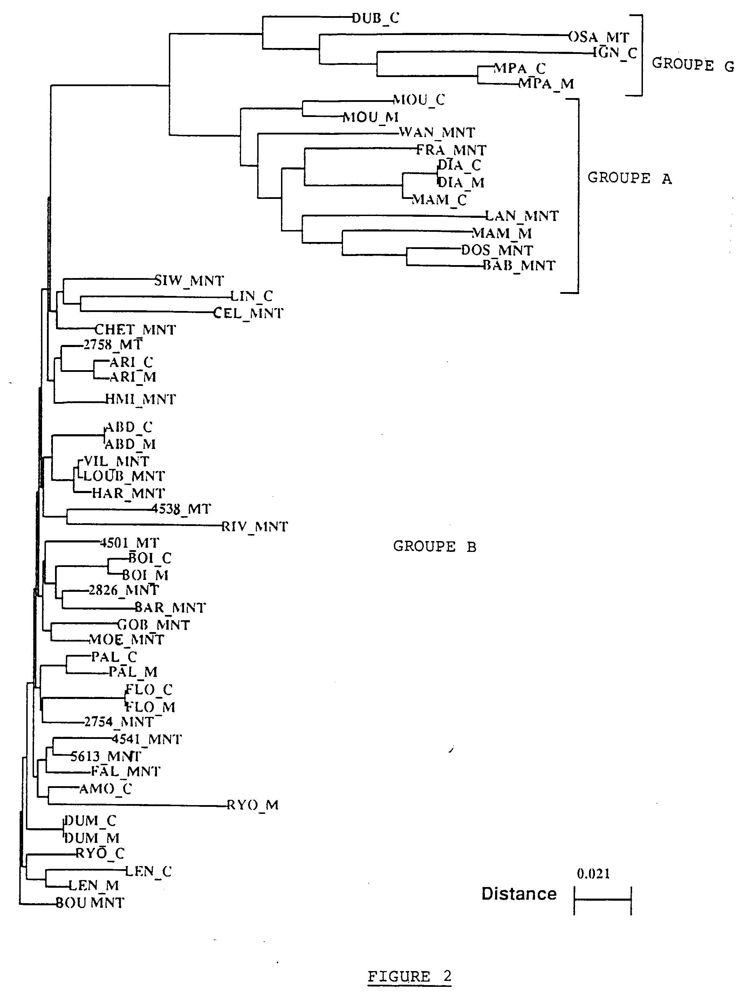

A la figure 2, un arbre phylogénique montre la distribution des séquences d'ADN consensus dans plusieurs clades. Dans presque toutes les paires mère-enfant, la séquence de l'isolat retrouvé chez l'enfant est plus proche de la séquence de l'isolat de sa mère que de n'importe quelle autre séquence montrant le lien génétique existant dans chaque paire.

La figure 3 montre l'alignement de 54 séquences peptidiques obtenues.

Dans la mesure où il n'y pas eu d'étape de clonage, chaque séquence d'isolats constitue le consensus des séquences variantes majeures.

Pour 4 enfants et 8 mères, les séquences d'isolats étaient hétérogènes, au niveau de 1 à 8 localisations (acide aminé noté ? dans les séquences de la figure 2).

Pour les autres isolats, une séquence unique p17 a été observée.

Chaque séquence d'isolats d'enfant est très proche de celle de la mère (0,5 à 2 %), suggérant que le virus transmis n'est pas un variant mineur.

Les sous-types viraux de l'enfant sont toujours retrouvés dans les isolats de la mère analysés préalablement, conformément aux résultats obtenus sur le séquençage direct de la région *env* V3-V5 et d'autres études qui ont effectué le sous-clonage du gène *env.*

De plus, comme dans le cas de la boucle V3, dans laquelle une variation intrapatient faible est observée pour les enfants pendant le premier mois de la vie, la p17 ne semble pas varier pendant cette période de temps.

Les séquences peptidiques des isolats ont été classées parmi les différents sous-types de VIH-1, A, B, G et D.

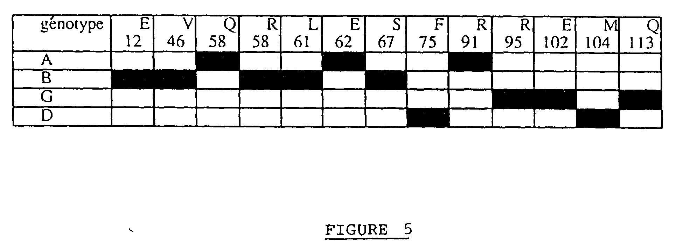

Conformément à MYERS (Human retroviruses and AIDS compendium Los Alamos, N.M, 1994), certains aminoacides peuvent être considérés comme une signature de chaque sous-type (figure 3 et figure 5).

Cette subdivision est presque identique aux clades de séquences d'ADN, à l'exception de l'urique isolat du sous-type D.

Cette division en sous-types a permis d'éliminer toutes les variations de séquences dues aux sous-types. Lorsque l'on compare les domaines fonctionnels de toutes les protéines p17 séquencées, aucune différence significative n'apparaît ni au niveau du site de myristoylation, ni au niveau des sites de localisation nucléaire.

Cependant, un groupe de variation spécifique est observé au niveau du site de polymérisation p17 (de l'aminoacide 47 Asn à l'aminoacide 59 Gln) et dans les sites de phosphorylation (aminoacides 110-114, qui sont respectivement Lys Ser Lys Gln Lys, Lys Ser Lys Lys Lys et Ile Ser GIn Gln Lys dans les sous-types A, B et G). Le site de phosphorylation de la tyrosine Y132 décrit par GALLAY et al. (Cell, 1995, **80**, 379-388) est conservé dans tous les sous-types.

Deux domaines présentant une variabilité importante sont observés entre les aminoacides 75 et 95 et entre les aminoacides 103 et 109.

Les variations dans la première région ne sont pas associées avec les sous-types et ne sont pas non plus associées avec la transmission.

De manière surprenante, lorsque l'on étudie de manière plus précise le domaine 103-109, en particulier dans le sous-type B, le motif Lys Ile Glu Glu Glu Gln Asn se retrouve de manière constante chez tous les enfants auxquels le virus a été transmis.

De plus, ce motif est constant dans les isolats des mères transmettrices.

A l'opposé, parmi les 16 virus non transmis, seulement 8 présentent le motif Lys Ile Glu Glu Glu Gln Asn.

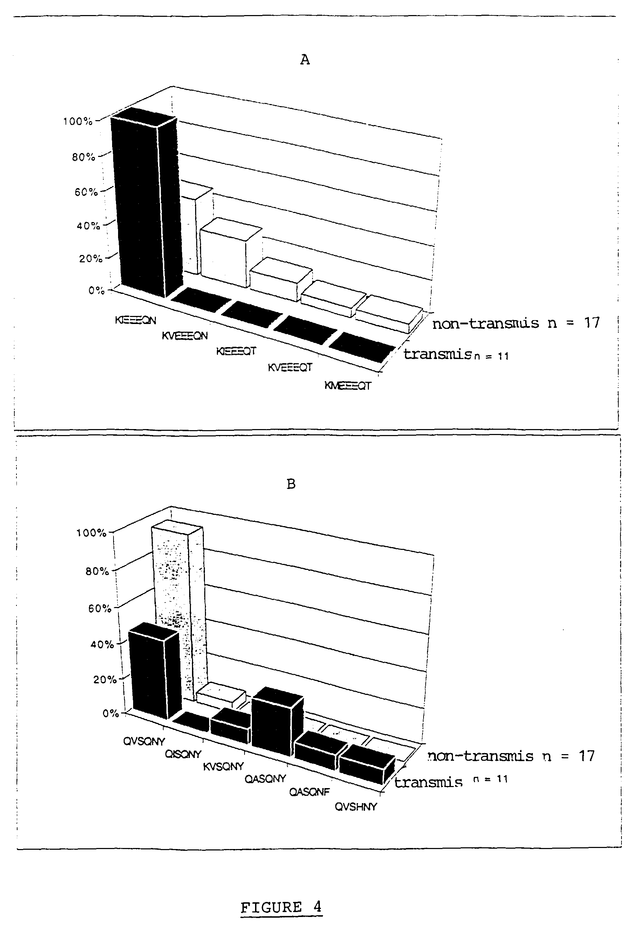

5 des virus non transmis ont un motif Lys Val Glu Glu Glu Gln Asn, 2 un motif Lys Ile Glu Glu Glu Gln Asn et 1 un motif Lys Val Glu Glu Glu Gln Thr (figure 4A et figure 4B).

La moitié N-terminale de la p17 est globalement conservée dans chaque sous-type. A l'opposé, plusieurs domaines de variabilité sont observés dans la portion C-terminale de la p17.

Ces domaines de variabilité et plusieurs aminoacides variables ont été comparés dans le sous-groupe B, qui constitue le sous-groupe le plus important de la cohorte étudiée.

Ces comparaisons ont été réalisées entre les groupes non-transmetteurs et les groupes transmetteurs. Ces résultats sont illustrés à la figure 4A qui montre la relation entre la séquence 103-109 de la protéine p17 de VIH-1 et la transmission observée chez l'enfant dans une cohorte de mères infectées par un VIH-1 de sous-groupe B. Le pourcentage de chaque motif 103-109 est indiqué en gris pour les mères non-transmettrices et en noir pour les mères transmettrices.

En particulier, toutes les séquences transmissibles comprennent en tant que séquence X2 X3 Glu X4 X5 X6 X7, la séquence Lys Ile Glu Glu Glu Gln Asn (motif KIEEQN) (*X*² = p=0,0034), alors que la présence d'une valine pour X3 est associée de manière significative à un phénotype non-transmissible (*X*² = p=0,014).

Le Tableau II ci-après correspond à une analyse statistique des aminoacides et des motifs variables de la protéine p17 du sous-type VIH-1 B.

**TABLEAU II**

| Aminoacides | Nombre dans 11 isolats transmis | Nombre dans 17 isolats non-transmis | *X*² | *X*² corrigé pour continuité |
|---|---|---|---|---|
| Arginine 30 | 6 | 8 | 0,6957 | 1 |
| Arginine 75 | 7 | 8 | 0,395 | 0,637 |
| Acide glutamique 93 | 9 | 7 | 0,0338 | 0,083 |
| Acide glutamique 102 | 6 | 14 | 0,1117 | 0,245 |
| KIEEEQN | 11 | 8 | 0,0034 | 0,012 |
| Valine 104 | 0 | 7 | 0,014 | 0,044 |
| Glycine 120 | 8 | 11 | 0,657 | 0,976 |
| QVSQNY | 5 | 16 | 0,0037 | 0,014 |

2 aminoacides et 2 domaines semblent être suffisants en eux-mêmes pour discriminer le groupe non-transmetteur du groupe transmetteur :
1) Le résidu acide glutamique, en position 93 est associé de manière significative avec le phénotype transmetteur (*X*² p = 0,0338).
2) Le premier domaine statistiquement significatif pour l'analyse de la transmission est le motif QVSQNY correspondant au fragment C-terminal de la protéine p17 ; en particulier, dans le sous-type B, il est associé de manière significative avec le groupe non-transmetteur (*X*² p = 0,0037). Ces résultats sont illustrés à la figure 4B qui montre la relation entre les séquences C-terminales de la protéine HIV-1 p17 et la transmission du virus dans la même cohorte de mères. Il ressort de ces résultats que le motif QVSQNY est associé à une absence de transmission.
3) Selon la présence du motif Lys Ile Glu Glu Glu Gln Asn, il y a une différence significative entre les isolats de mères transmettrices et les isolats de mères non transmettrices (*X*² = p = 0,0034).

De plus, les 6 virus transmis du sous-type B présentent le même motif peptidique Lys Ile Glu Glu Glu Gln Asn.

4) En outre, la présence du résidu valine en position 104 semble être associée avec le phénotype non transmetteur (*X*² = p=0,014).

En conséquence, la méthode selon l'invention qui comprend un ensemble de peptides permet effectivement d'évaluer le risque de transmission materno-foetale du VIH-1 et de ce fait, permet d'éliminer les cas sûrs et/ou douteux de transmission (figure 5).

### EXEMPLE 4 : Préparation d'anticorps témoins.

### 1) Préparation des peptides :

La synthèse chimique des peptides cibles est effectuée sur un synthétiseur 433 (Applied biosystem) utilisant la chimie FMOC, le suivi de synthèse est réalisé par conductimétrie et les peptides purifiés par HPLC semi-préparative. 15 mg de chacun des peptides peuvent être produits avec une pureté de 98 à 99 %.

Deux résidus (Tyr-Gly) ont été ajoutés à l'extrémité N-terminale pour les besoins de synthèse.

6,7 mg de chacun de ces peptides ont été couplés à 10 mg de protéine KLH (réactif BDB). Les différents conjugués obtenus sont utilisés pour préparer des anticorps spécifiques.

### 2) Préparation des anticorps témoins :

150 µg de chaque peptide tel qu'obtenu en 1) sont utilisés pour préparer des anticorps IgY spécifiques, par immunisation de poule par la société BioCytex-bioService (Marseille, France) suivant le protocole suivant :
phase d'immunisation :
- J1 :: Contrôle pré-immun sur sérum ou sur oeuf
Injection intra-musculaire de l'antigène en adjuvant complet de Freund
- J10 :: Injection intramusculaire de l'antigène en adjuvant complet de Freund
Collecte des oeufs de J11 à J19 et contrôle de la réponse immunitaire par méthode ELISA
- J20 :: Injection intra-musculaire de l'antigène en adjuvant complet de Freund
phase de production
A partir de J21: collecte de 6 à 7 oeufs
- J28 :: Extraction des immunoglobulines Y
Détermination de la concentration en Ig Y spécifique par méthode ELISA
De l'immun-sérum sera aussi prélevé.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Fragment d'acide nucléique, issu du gène codant pour un fragment de la protéine de matrice p17 du VIH-1, essentiellement constitué de 21 à 90 nucléotides et incluant au moins la séquence représentée par la formule : Y5 Y6 Y7 Y8 Y9 Y10 Y11, dans laquelle :
Y5 représente AAA, AAG, GAA,
Y6 représente ATA, TTA, CTG, GTA, CTA, GTG, ATG,
Y7 représente GAG, GAA,
Y8 représente GAA, AAG,
Y9 représente ATA, GTA, CTA, GAA, GAG, GAC, GAT, AGA,
Y10 représente CAA, CAG, CGA,
Y11 représente AAA, AGT, AAG, AAT, CAT, AAC, ACC,
à condition que lorsque Y5 représente AAA ou AAG, Y6 soit différent de ATA, Y7 soit différent de GAG ou GAA, Y8 soit différent de GAA, Y9 soit différent de GAG ou GAA, Y10 soit différent de CAA ou CAG et Y11 soit différent de AAT ou AAC.

2. Fragment d'acide nucléique selon la revendication 1, caractérisé en ce qu'il présente la formule suivante :
Y1 GAA Y2 Y3 Y4 Y5 Y6 Y7 Y8 Y9 Y10 Y11 Y12 Y13 Y14 Y15 Y16 Y17
dans laquelle
Y5-Y11 ont la même signification que ci-dessus et
Y1 représente AAA, AAG,
Y2 représente GCT, GCC,
Y3 représente TTA, CTA,
Y4 représente GAT, GAC, GAG,
Y12 représente AGG, AAG, AAA, ATC, AAC,
Y13 représente AGC, AAC, AGT, AAG,
Y14 représente GGG, AAG, AAA, CAG,
Y15 représente CAA, AAA, AGA,
Y16 représente AAG, AAA, ACA, CAA, AAC, AAT,
Y17 représente ACA, GCA, AGC, ATA, GCC, GCT, GCG.

3. Fragment d'acide nucléique selon la revendication 1 ou la revendication 2, caractérisé en ce qu'il est choisi dans le groupe constitué par les séquences suivantes : SEQ ID N° 1 (DIA), SEQ ID N° 2 (MAM), SEQ ID N° 3 (MOU), SEQ ID N° 4 (BAB), SEQ ID N° 5 (DOS), SEQ ID N° 6 (LAN), SEQ ID N° 7 (FRA), SEQ ID N° 8 (WAN), SEQ ID N° 9 (ABD), SEQ ID N° 10 (ARI), SEQ ID N° 11 (BOI), SEQ ID N° 12 (DUM), SEQ ID N° 13 (PAL), SEQ ID N° 14 (RYO), SEQ ID N° 15 (FLO), SEQ ID N° 16 (4501), SEQ ID N° 17 (FAL), SEQ ID N° 18 (HAR), SEQ ID N° 19 (RIV), SEQ ID N° 20 (CHET), SEQ ID N° 21 (CEL), SEQ ID N° 22 (SIW), SEQ ID N° 23 (MOE), SEQ ID N° 24 (GOB), SEQ ID N° 25 (4541), SEQ ID N° 26 (2754), SEQ ID N° 27 (2826), SEQ ID N° 28 (MPA), SEQ ID N° 29 (IGN), SEQ ID N° 30 (OSA) et SEQ ID N° 31 (BOU) ou un fragment de celles-ci, tel que : SEQ ID N° 32 (sonde 1): SEQ ID N° 33 (sonde 2), SEQ ID N° 34 (sonde 3), SEQ ID N° 35 (sonde 4), SEQ ID N°56 (sonde 5) et SEQ ID N°57 (sonde 6).

4. Peptides, constitués de 7 à 30 amino-acides et incluant au moins la séquence en aminoacides 103 à 109 de la protéine de matrice virale p17 du VIH- 1, représentée par la formule X2 X3 Glu X4 X5 X6 X7, dans laquelle :
X2 représente Lys, Glu
X3 représente Ile, Leu, Val, Met
X4 représente Glu, Lys
X5 représente Ile, Val, Glu, Arg, Leu
X6 représente Gln, Arg et
X7 représente Lys, Ser, Asn, His, Thr,
à condition que lorsque X2 représente Lys, X3 soit différent de Ile, X4 et X5 soient différents de Glu, X6 soit différent de Gln et X7 soit différent de Asn.

5. Peptide selon la revendication 4, caractérisé en ce qu'il présente la formule suivante :
Lys Glu Ala Leu X1 X2 X3 Glu X4 X5 X6 X7 X8 X9 X10 X11 X12 X13,
dans laquelle
X2-X7 ont la même signification que ci-dessus et
X1 représente Asp, Glu
X8 représente Arg, Lys, Ile, Asn
X9 représente Ser, Asn, Lys
X10 représente Gly, Lys, Gln, Glu
X11 représente Gln, Lys, Arg
X12 représente Lys, Gln
X13 représente Thr, Ala, Ile.

6. Peptide selon la revendication 4 ou la revendication 5, caractérisé en ce qu'il est sélectionné parmi les peptides suivants : SEQ ID N° 36 (DIA), SEQ ID N° 37 (MAM), SEQ ID N° 38 (MOU), SEQ ID N° 39 (BAB), SEQ ID N° 40 (DOS), SEQ ID N° 41 (LAN), SEQ ID N° 42 (FRA), SEQ ID N° 43 (WAN), SEQ ID N° 44 (ABD), SEQ ID N° 45 (BOI), SEQ ID N° 46 (4501), SEQ ID N° 47 (HAR), SEQ ID N° 48 (CEL), SEQ ID N° 49 (SIW), SEQ ID N° 50 (MOE), SEQ ID N° 51 (GOB), SEQ ID N° 52 (MPA), SEQ ID N° 53 (IGN), SEQ ID N° 54 (OSA), SEQ ID N° 55 (BOU).

7. Anticorps dirigés contre les peptides selon l'une quelconque des revendications 4 à 6 et le peptide dans lequel X2 représente Lys, X3 représente Ile, X4 et X5 représentent Glu, X6 représente Gln et X7 représente Asn.

8. Anticorps selon la revendication 7, caractérisés en ce qu'ils consistent en anticorps polyclonaux et en ce qu'ils sont obtenus par immunisation d'un animal approprié avec un peptide selon la revendication 4, la revendication 5 ou la revendication 6 ou un peptide dans lequel X2 représente Lys, X3 représente Ile, X4 et X5 représentent Glu, X6 représente Gln et X7 représente Asn, éventuellement couplé à une protéine convenable telle que la sérum albumine bovine ou la KLH.

9. Anticorps selon la revendication 7, caractérisés en ce qu'ils consistent en anticorps monoclonaux et en ce qu'ils sont obtenus par fusion de cellules spléniques de souris immunisées avec un antigène consistant en un fragment peptidique selon l'une quelconque des revendications 4 à 6 ou un peptide dans lequel X2 représente Lys, X3 représente Ile, X4 et X5 représentent Glu, X6 représente Gln et X7 représente Asn, éventuellement couplé à une protéine convenable telle que la SAB ou la KLH, avec des cellules myélomateuses appropriées.

10. Réactif pour le dépistage et l'évaluation du risque de transmission materno-foetale du VIH-1, caractérisé en ce qu'il est sélectionné dans le groupe constitué par les séquences nucléotidiques selon l'une quelconque des revendications 1 à 3, la séquence nucléique dans laquelle Y5 représente AAA ou AAG, Y6 représente ATA, Y7 représente GAG ou GAA, Y8 représente GAA, Y9 représente GAG ou GAA, Y10 représente CAA ou CAG et Y11 représente AAT ou AAC, et les fragments peptidiques selon l'une quelconque des revendications 4 à 6 et le peptide dans lequel X2 représente Lys, X3 représente Ile, X4 et X5 représentent Glu, X6 représente Gln et X7 représente Asn, et les anticorps anti-peptides selon l'une quelconque des revendications 7 à 9.

11. Réactif selon la revendication 10, caractérisé en ce qu'il est constitué par un *pool* ou ensemble de peptides selon l'une quelconque des revendications 4 à 6 et un peptide dans lequel X2 représente Lys, X3 représente Ile, X4 et X5 représentent Glu, X6 représente Gln et X7 représente Asn,.

12. Réactif selon la revendication 10, caractérisé en ce qu'il est constitué par un *pool* ou ensemble d'anticorps anti-peptidiques selon l'une quelconque des revendications 7 à 9.

13. Réactif selon la revendication 10, caractérisé en ce que lorsqu'il est constitué par au moins une séquence d'acide nucléique, il comprend au moins une séquence codant pour les amino-acides 103-109 de la protéine p17 du VIH-1, représentée par la formule Y5 Y6 Y7 Y8 Y9 Y10 Y11, dans laquelle :
Y5 représente AAA, AAG, GAA,
Y6 représente ATA, TTA, CTG, GTA, CTA, GTG, ATG,
Y7 représente GAG, GAA,
Y8 représente GAA, AAG,
Y9 représente ATA, GTA, CTA, GAA, GAG, GAC, GAT, AGA,
Y10 représente CAA, CAG, CGA,
Y11 représente AAA, AGT, AAG, AAT, CAT, AAC ou
il est apte à détecter spécifiquement une séquence nucléique contenant ladite formule.

14. Réactif selon la revendication 13, caractérisé en ce qu'il est notamment sélectionné dans le groupe constitué par les séquences nucléotidiques ou les fragments de celles-ci selon l'une quelconque des revendications 1 à 3 et la séquence nucléique dans laquelle Y5 représente AAA ou AAG, Y6 représente ATA, Y7 représente GAG ou GAA, Y8 représente GAA, Y9 représente GAG ou GAA, Y10 représente CAA ou CAG et Y11 représente AAT ou AAC, associées à au moins un moyen de détection approprié.

15. Réactif selon la revendication 13 ou la revendication 14, caractérisé en ce qu'il est constitué par l'un des fragments suivants: SEQ ID N° 32 (sonde 1), SEQ ID N° 33 (sonde 2), SEQ ID N° 34 (sonde 3), SEQ ID N° 35 (sonde 4), SEQ ID N° 56 (sonde 5) et SEQ ID N° 57 (sonde 6).

16. Réactif selon la revendication 10, caractérisé en ce que lorsqu'il est constitué d'au moins un peptide, il comprend au moins une séquence en amino-acides 103-109 de la protéine p17 du VIH-1, représentée par la formule X2 X3 Glu X4 X5 X6 X7, dans laquelle :
X2 représente Lys, Glu
X3 représente Ile, Leu, Val, Met
X4 représente Glu, Lys
X5 représente Ile, Val, Glu, Arg, Leu,
X6 représente Gln, Arg et
X7 représente Lys, Ser, Asn, His, Thr ou
il est apte à détecter spécifiquement un peptide contenant ladite formule.

17. Procédé de détection, de dépistage et d'évaluation du risque de transmission materno-foetale du VIH-1, dans un échantillon biologique, caractérisé en ce qu'il comprend une étape dans laquelle l'on met en contact l'échantillon biologique avec un réactif selon l'une quelconque des revendications 10 à 16 et une étape dans laquelle on détecte une interaction spécifique entre ledit réactif et une ou plusieurs séquences de l'ADN dudit virus, consistant en l'une des séquences d'acide nucléique selon l'une quelconque des revendications 1 à 3 ou la séquence nucléique dans laquelle Y5 représente AAA ou AAG, Y6 représente ATA, Y7 représente GAG ou GAA, Y8 représente GAA, Y9 représente GAG ou GAA, Y10 représente CAA ou CAG et Y11 représente AAT ou AAC et présentes dans ledit échantillon biologique.

18. Procédé selon la revendication 17, caractérisé en ce que la détection de l'une des séquences selon l'une quelconque des revendications 1 à 3 ou la séquence nucléique dans laquelle Y5 représente AAA ou AAG, Y6 représente ATA, Y7 représente GAG ou GAA, Y8 représente GAA, Y9 représente GAG ou GAA, Y10 représente CAA ou CAG et Y11 représente AAT ou AAC est effectuée après amplification par la méthode d'amplification PCR de la région codant pour la protéine de matrice virale p17, suivie de l'hybridation du produit obtenu, sur membrane sur laquelle ont préalablement été fixées une ou plusieurs des sondes selon l'une quelconque des revendications 13 à 15.

19. Kit, coffret ou ensemble coordonné prêt à l'emploi, pour la mise en oeuvre du procédé de détection, de dépistage et d'évaluation du risque de transmission materno-foetale du VIH-1 selon la revendication 17 ou la revendication 18, caractérisé en ce qu'il comprend outre des quantités de tampons et de réactifs appropriés pour la mise en oeuvre de ladite détection, des doses appropriées d'au moins une sonde ou fragment de sonde nucléotidique selon l'une quelconque des revendications 13 à 15.

20. Kit selon la revendication 19, caractérisé en ce qu'il comporte les éléments suivants :
- les réactifs nécessaires pour effectuer une amplification PCR d'ADN,
- des doses appropriées d'amorces spécifiques de la région p17 du gène gag du VIH-1, et
- au moins une séquence nucléotidique selon l'une quelconque des revendications 13 à 15, capable de s'hybrider, dans des conditions stringentes à la partie de la séquence de la région p17 du gène gag du VIH-1 comprenant l'une des séquences nucléotidiques selon la revendication 1, 2 ou 3 ou la séquence nucléique dans laquelle Y5 représente AAA ou AAG, Y6 représente ATA, Y7 représente GAG ou GAA, Y8 représente GAA, Y9 représente GAG ou GAA, Y10 représente CAA ou CAG et Y11 représente AAT ou AAC.

21. Procédé de détection, de dépistage et d'évaluation du risque de transmission materno-foetale du VIH-1, caractérisé en ce qu'il consiste à détecter l'une des séquences peptidiques selon les revendications 4 à 6 ou une séquence peptidique dans laquelle X2 représente Lys, X3 représente Ile, X4 et X5 représentent Glu, X6 représente Gln et X7 représente Asn, dans un échantillon biologique maternel, en mettant en présence ledit échantillon biologique avec un *pool* d'anticorps spécifiques desdites différentes séquences peptidiques selon l'une quelconque des revendications 7 à 9.

22. Procédé de détection, de dépistage et d'évaluation du risque de transmission materno-foetale du VIH-1, caractérisé en ce qu'il consiste à détecter l'un des anticorps anti-peptides selon l'une quelconque des revendications 7 à 9, dans un échantillon biologique maternel, en mettant en présence ledit échantillon biologique avec un *pool* de peptides selon les revendications 4 à 6 et la séquence peptidique dans laquelle X2 représente Lys, X3 représente Ile, X4 et X5 représentent Glu, X6 représente Gln et X7 représente Asn,.

23. Procédé selon la revendication 22, caractérisé en ce que ledit *pool* de peptides est constitué des séquences ID N° 44, 45, 47, 48, 50 et 51.

24. Kit, coffret ou ensemble coordonné prêt à l'emploi, pour la mise en oeuvre du procédé de détection, de dépistage et d'évaluation du risque de transmission materno-foetale du VIH-1, selon la revendication 21 ou la revendication 22, caractérisé en ce qu'il comprend outre des quantités de tampons et de réactifs appropriés pour la mise en oeuvré de ladite détection, un support solide approprié convenablement revêtu d'au moins un ligand choisi dans le groupe qui comprend les peptides selon les revendications 4 à 6 ou le peptide dans lequel X2 représente Lys, X3 représente Ile, X4 et X5 représentent Glu, X6 représente Gln et X7 représente Asn, et les anticorps selon l'une quelconque des revendications 7 à 9, au moins un flacon contenant des conjugués choisis dans le groupe qui comprend les conjugués enzyme appropriée-anticorps anti-Ig humains appropriés, de chèvre notamment, et les conjugués enzyme appropriée-anticorps anti-peptides selon l'une quelconque des revendications 7 à 9 et des quantités ou doses appropriées d'une substance de révélation appropriée.


LISTE DE SEQUENCES

(1) INFORMATIONS GENERALES:

(i) DEPOSANT:
(A) NOM: COMMISSARIAT A L'ENERGIE ATOMIQUE
(B) RUE: 31-33 RUE DE LA FEDERATION
(C) VILLE: PARIS
(E) PAYS: FRANCE
(F) CODE POSTAL: 75015

(ii) TITRE DE L' INVENTION: FRAGMENTS D'ACIDE NUCLEIQUE DERIVES
DU GENOME DU VIRUS VIH-1, PEPTIDES CORRESPONDANTS ET
LEURS APPLICATIONS EN TANT QUE REACTIFS D'EVALUATION DU
RISQUE DE TRANSMISSION MATERNO-FOETALE DU VIH-1.

(iii) NOMBRE DE SEQUENCES: 57

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 1:

AAAGAAGCTT TAGATAAAAT AGAGGAAATA CAAAAAAGGA GCGGGCAAAA GACA 54

(2) INFORMATIONS POUR LA SEQ ID NO: 2:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 2:

AAAGAAGCCT TAGACAAAAT AGAGGAAATA CAAAGTAAGA ACAAGCAAAA GGCA 54

(2) INFORMATIONS POUR LA SEQ ID NO: 3:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 3:

AAGGAAGCTT TAGATAAAAT AGAGGAAGTA CAGAAAAAGA GCAAGCAAAA GACA 54

(2) INFORMATIONS POUR LA SEQ ID NO: 4:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 4:

AAGGAAGCCT TAGATAAAAT AGAGGAAATA CAAAAGAAGA GCAAGCAAAA GACA 54

(2) INFORMATIONS POUR LA SEQ ID NO: 5:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 5:

AAGGAAGCTT TAGATAAATT AGAGGAAATA CAAAATAAGA GCAAACAAAA GACA 54

(2) INFORMATIONS POUR LA SEQ ID NO: 6:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 6:

AAGGAAGCTT TAGATAAGCT GGAGGAAATA CAACATAAGA ACAAGCAAAA GACA 54

(2) INFORMATIONS POUR LA SEQ ID NO: 7:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 7:

AAGGAAGCCT TAGATAAAAT AGAGGAGATA CAAAATAAGA GCAAGCAAAA AACA 54

(2) INFORMATIONS POUR LA SEQ ID NO: 8:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 8:

AAGGAAGCTT TAGATAAAAT AGAGGAACTA CAAAAGAAGA GTAAGCAAAA AGCA 54

(2) INFORMATIONS POUR LA SEQ ID NO: 9:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 9:

AAGGAAGCTT TAGATAAGAT AGAGGAAGAA CAAAACAAAA GTAAGAAAAA AGCA 54

(2) INFORMATIONS POUR LA SEQ ID NO: 10:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 10:

AAGGAAGCTT TAGATAAGAT AGAGGAAGAG CAAAACAAAA GTAAGAAAAA AGCA 54

(2) INFORMATIONS POUR LA SEQ ID NO: 11:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 11:

AAGGAAGCTT TAGAGAAGAT AGAGGAAGAG CAAAACAAAA GTAAGAAAAA GGCA 54

(2) INFORMATIONS POUR LA SEQ ID NO: 12:

(i) CARACTERISTIQUES DE LA SEQUENCE:
(A) LONGUEUR: 54 paires de bases

(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 12:

AAGGAAGCTT TAGAGAAGAT AGAGGAAGAG CAAAACAAAA GTAAGAAAAA AGCA 54

(2) INFORMATIONS POUR LA SEQ ID NO: 13:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 13:

AAGGAAGCTT TAGATAAGAT AGAGGAAGAG CAAAACAAAA GTAAGAAAAA AGCA 54

(2) INFORMATIONS POUR LA SEQ ID NO: 14:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 14:

AAGGAAGCTT TAGACAAGAT AGAAGAAGAG CAAAACAAAA GTAAGAAAAA AGCA 54

(2) INFORMATIONS POUR LA SEQ ID NO: 15:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 15:

AAGGAAGCTT TAGAGAAGAT AGAGGAAGAG CAAAACAAAA GTAAGAAAAC AAGC 54

(2) INFORMATIONS POUR LA SEQ ID NO: 16:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 16:

AAGGAAGCTT TAGAGAAGAT AGAGGAAGAG CAAAACAAAA GTAAGAAACA AGCA 54

(2) INFORMATIONS POUR LA SEQ ID NO: 17:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 17:

AAGGAAGCTT TAGAGAAGAT AGAGGAAGAG CAAAACAAAA GTAAGAAAAA AGCA 54

(2) INFORMATIONS POUR LA SEQ ID NO: 18:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 18:

AAGGAAGCTC TAGAGAAGGT AGAGGAAGAG CAAAACAAAA GTAAGAAAAA NGCA 54

(2) INFORMATIONS POUR LA SEQ ID NO: 19:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 19:

AAGGAAGCTT TAGACAAGAT AGAGGAAGAA CAAAACAAAA GTAAGAAAAA AGCA 54

(2) INFORMATIONS POUR LA SEQ ID NO: 20:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 20:

AAGGAAGCTT TAGAGAAGAT AGAGGAAGAG CAAAACAAAA GTAAGAAAAA GGCA 54

(2) INFORMATIONS POUR LA SEQ ID NO: 21:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 21:

AAGGAAGCTT TAGAGAAGGT AGAGGAAGAG CAAACCAAAA GTAAGAGAAA AGCA 54

(2) INFORMATIONS POUR LA SEQ ID NO: 22:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 22:

AAGGAAGCTC TAGACAAGAT AGAGGAAGAG CAAAACAAAA GTAAGAAAAA GGCA 54

(2) INFORMATIONS POUR LA SEQ ID NO: 23:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 23:

AAGGAAGCTT TAGACAAGAT AGAGGAAGAG CAAACCAAAA GTAAGAAAAA AGCA 54

(2) INFORMATIONS POUR LA SEQ ID NO: 24:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 24:

AAGGAAGCTT TAGAGAAGAT AGAGGAAGAN CAAACCAAAA GTAAGAAAAA AGCA 54

(2) INFORMATIONS POUR LA SEQ ID NO: 25:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 25:

AAGGAAGCTT TAGAGAAAGT AGAGGAAGAG CAAAACAAAA GTAAGAAAAA GGCA 54

(2) INFORMATIONS POUR LA SEQ ID NO: 26:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 26:

AAGGAAGCTT TAGAGAAGGT AGAGGAAGAG CAAAACAAAA GTAAGAAAAA AGCA 54

(2) INFORMATIONS POUR LA SEQ ID NO: 27:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 27:

AAGGAAGCTT TAGAGAAGAT AGAGGAAGAG CAAAACAAGA GTAAGAAAAA AGCA 54

(2) INFORMATIONS POUR LA SEQ ID NO: 28:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 28:

AAAGAAGCTC TAGAGGAAGT GGAAAAGAGA CAAAAGATCA GTCAGCAAAA AATA 54

(2) INFORMATIONS POUR LA SEQ ID NO: 29:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 29:

AAAGAAGCTC TAGAGGAAGT GGAAAAGAGA CAAAAGATCA GTCAGAAAAA AATA 54

(2) INFORMATIONS POUR LA SEQ ID NO: 30:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 30:

AAAGAAGCTC TAGAGGAAGT GGAAAAGGTA CGAAAAAACA AGCAGCAAAA AGCA 54

(2) INFORMATIONS POUR LA SEQ ID NO: 31:

(i) CARACTERISTIQUES DE LA SEQUENCE:
(A) LONGUEUR: 54 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 31:

AAGGAAGCTT TAGAGAAGAT GGAGGAAGAG CAAAACAAAA GTAAGAAAAA AGCA 54

(2) INFORMATIONS POUR LA SEQ ID NO: 32:

(i) CARACTERISTIQUES DE LA SEQUENCE:
(A) LONGUEUR: 25 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "sonde"

(xi) DESCRIPTION DE LA SEQUENCE: SEQ ID NO: 32:

TTTTGTTTTG CTCTTCCTCT ATCTT 25

(2) INFORMATIONS POUR LA SEQ ID NO: 33:

(i) CARACTERISTIQUES DE LA SEQUENCE:
(A) LONGUEUR: 19 paires de bases

(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "sonde"

(xi) DESCRIPTION DE LA SEQUENCE: SEQ ID NO: 33:

GTTTTGCTCT TCCTCTACC 19

(2) INFORMATIONS POUR LA SEQ ID NO: 34:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "sonde"

(xi) DESCRIPTION DE LA SEQUENCE: SEQ ID NO: 34:

GGTTTGCTCT TCCTCTATC 19

(2) INFORMATIONS POUR LA SEQ ID NO: 35:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "sonde"

(xi) DESCRIPTION DE LA SEQUENCE: SEQ ID NO: 35:

GTTTGCTCTT CCTCTACCT 19

(2) INFORMATIONS POUR LA SEQ ID NO: 36:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 36:

Lys Glu Ala Leu Asp Lys Ile Glu Glu Ile Gln Lys Arg Ser Gly Gln
1               5                   10                  15

Lys Thr

(2) INFORMATIONS POUR LA SEQ ID NO: 37:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 37:

Lys Glu Ala Leu Asp Lys Ile Glu Glu Ile Gln Ser Lys Asn Lys Gln
1               5                   10                  15

Lys Ala

(2) INFORMATIONS POUR LA SEQ ID NO: 38:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 38:

Lys Glu Ala Leu Asp Lys Ile Glu Glu Val Gln Lys Lys Ser Lys Gln
1               5                   10                  15

Lys Thr

(2) INFORMATIONS POUR LA SEQ ID NO: 39:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 39:

Lys Glu Ala Leu Asp Lys Ile Glu Glu Ile Gln Lys Lys Ser Lys Gln
1               5                   10                  15

Lys Thr

(2) INFORMATIONS POUR LA SEQ ID NO: 40:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 40:

Lys Glu Ala Leu Asp Lys Leu Glu Glu Ile Gln Asn Lys Ser Lys Gln
1               5                   10                  15

Lys Thr

(2) INFORMATIONS POUR LA SEQ ID NO: 41:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 41:

Lys Glu Ala Leu Asp Lys Leu Glu Glu Ile Gln His Lys Asn Lys Gln
1               5                   10                  15

Lys Thr

(2) INFORMATIONS POUR LA SEQ ID NO: 42:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 42:

Lys Glu Ala Leu Asp Lys Ile Glu Glu Ile Gln Asn Lys Ser Lys Gln
1               5                   10                  15

Lys Thr

(2) INFORMATIONS POUR LA SEQ ID NO: 43:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 43:

Lys Glu Ala Leu Asp Lys Ile Glu Glu Leu Gln Lys Lys Ser Lys Gln
1               5                   10                  15

Lys Ala

(2) INFORMATIONS POUR LA SEQ ID NO: 44:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 44:

Lys Glu Ala Leu Asp Lys Ile Glu Glu Glu Gln Asn Lys Ser Lys Lys
1               5                   10                  15

Lys Ala

(2) INFORMATIONS POUR LA SEQ ID NO: 45:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 45:

Lys Glu Ala Leu Glu Lys Ile Glu Glu Glu Gln Asn Lys Ser Lys Lys
1               5                   10                  15

Lys Ala

(2) INFORMATIONS POUR LA SEQ ID NO: 46:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 46:

Lys Glu Ala Leu Glu Lys Ile Glu Glu Glu Gln Asn Lys Ser Lys Lys
1               5                   10                  15

Gln Ala

(2) INFORMATIONS POUR LA SEQ ID NO: 47:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 47:

Lys Glu Ala Leu Glu Lys Val Glu Glu Glu Gln Asn Lys Ser Lys Lys
1               5                   10                  15

Lys Ala

(2) INFORMATIONS POUR LA SEQ ID NO: 48:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 48:

Lys Glu Ala Leu Glu Lys Val Glu Glu Glu Gln Thr Lys Ser Lys Arg
1               5                   10                  15

Lys Ala

(2) INFORMATIONS POUR LA SEQ ID NO: 49:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 49:

Lys Glu Ala Leu Glu Lys Ile Glu Glu Glu Gln Asn Lys Ser Glu Lys
1               5                   10                  15

Lys Ala

(2) INFORMATIONS POUR LA SEQ ID NO: 50:

(i) CARACTERISTIQUES DE LA SEQUENCE:
(A) LONGUEUR: 18 acides aminés
(B) TYPE: acide aminé
(C) NOMBRE DE BRINS: simple

(D) CONFIGURATION: linéaire

(ii) TYPE DE MOLECULE: peptide

(xi) DESCRIPTION DE LA SEQUENCE: SEQ ID NO: 50:

Lys Glu Ala Leu Asp Lys Ile Glu Glu Glu Gln Thr Lys Ser Lys Lys
1               5                   10                  15

Lys Ala

(2) INFORMATIONS POUR LA SEQ ID NO: 51:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 51:

Lys Glu Ala Leu Glu Lys Ile Glu Glu Glu Gln Thr Lys Ser Lys Lys
1               5                   10                  15

Lys Ala

(2) INFORMATIONS POUR LA SEQ ID NO: 52:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 52:

Lys Glu Ala Leu Glu Glu Val Glu Lys Arg Gln Lys Ile Ser Gln Gln
1               5                   10                  15

Lys Ile

(2) INFORMATIONS POUR LA SEQ ID NO: 53:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 53:

Lys Glu Ala Leu Glu Glu Val Glu Lys Arg Gln Lys Lys Ser Lys Gln
1               5                   10                  15

Lys Ile

(2) INFORMATIONS POUR LA SEQ ID NO: 54:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 54:

Lys Glu Ala Leu Glu Glu Val Glu Lys Val Arg Lys Asn Lys Gln Gln
1               5                   10                  15

Lys Ala

(2) INFORMATIONS POUR LA SEQ ID NO: 55:

(i) CARACTERISTIQUES DE LA SEQUENCE:
(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 55:

Lys Glu Ala Leu Glu Lys Met Glu Glu Glu Gln Asn Lys Ser Lys Lys
1               5                   10                  15

Lys Ala

(2) INFORMATIONS POUR LA SEQ ID NO: 56:

(i) CARACTERISTIQUES DE LA SEQUENCE:
(A) LONGUEUR: 25 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "sonde"

(xi) DESCRIPTION DE LA SEQUENCE: SEQ ID NO: 56:

TTTTGTTTTG CTCTTCCTCT ACTTT                                        25

(2) INFORMATIONS POUR LA SEQ ID NO: 57:

(i) CARACTERISTIQUES DE LA SEQUENCE:
(A) LONGUEUR: 25 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "sonde"

(xi) DESCRIPTION DE LA SEQUENCE: SEQ ID NO: 57:

CTTTTGTTTT GCTCTTCTTC TATCT 25